(19) Europäisches Patentamt
European Patent Office
Office européen des brevets

(11) EP 4 619 481 B1

(12) EUROPEAN PATENT SPECIFICATION

(45) Date of publication and mention of the grant of the patent:
**24.06.2026 Bulletin 2026/26**

(21) Application number: **24700958.2**

(22) Date of filing: **15.01.2024**

(51) International Patent Classification (IPC):
***C09D 183/04*** *(2006.01)* ***A61K 6/00*** *(2020.01)*

(52) Cooperative Patent Classification (CPC):
(C-Sets available)
**C09D 183/04; A61K 6/20; A61K 6/69** (Cont.)

(86) International application number:
**PCT/EP2024/050774**

(87) International publication number:
**WO 2024/153580 (25.07.2024 Gazette 2024/30)**

(54) **COATING COMPOSITION FOR THE TREATMENT OR PREVENTION OF PERIIMPLANTITIS AND PREPARATION METHOD THEREOF**

BESCHICHTUNGSZUSAMMENSETZUNG ZUR BEHANDLUNG ODER PRÄVENTION VON PERIIMPLANTITIS UND HERSTELLUNGSVERFAHREN DAFÜR

COMPOSITION DE REVÊTEMENT POUR LE TRAITEMENT OU LA PRÉVENTION DE LA PÉRIIMPLANTITE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Validation States:
**MA**

(30) Priority: **16.01.2023 EP 23382032**

(43) Date of publication of application:
**24.09.2025 Bulletin 2025/39**

(73) Proprietors:
- **Universitat Jaume I**
  **12071 Castelló de La Plana (Castellón) (ES)**
- **Universidad del País Vasco/Euskal Herriko Unibertsitatea**
  **48940 Leioa, Bikaia (ES)**

(72) Inventors:
- **SUAY ANTÓN, Julio José**
  **12071 Castelló de la Plana, Castellón (ES)**
- **ROMERO GAVILÁN, Francisco Javier**
  **12071 Castelló de la Plana, Castellón (ES)**
- **GURRUCHAGA TORRECILLA, María Dolores**
  **48940 Leioa, Vizcaya (ES)**
- **GOÑI ECHAVE, Isabel**
  **48940 Leioa, Vizcaya (ES)**
- **GARCÍA ARNÁEZ, Iñaki**
  **48940 Leioa, Vizcaya (ES)**

(74) Representative: **ABG Intellectual Property Law, S.L.**
**Avenida de Burgos, 16D**
**Edificio Euromor**
**28036 Madrid (ES)**

(56) References cited:
**US-A1- 2014 363 392**

- **GARCÍA-ARNÁEZ I. ET AL: "A single coating with antibacterial properties for prevention of medical device-associated infections", EUROPEAN POLYMER JOURNAL, vol. 113, 1 April 2019 (2019-04-01), GB, pages 289 - 296, XP093057411, ISSN: 0014-3057, DOI: 10.1016/ j.eurpolymj.2019.02.002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C09D 183/04, C08K 5/31**

## Description

### FIELD OF THE INVENTION

The present invention relates to a method for the preparation of a coating composition comprising an anti-infective agent for the controlled release of said anti-infective agent and to a coating composition or coated substrate obtainable by said method. Such coating compositions are particularly useful in the field of dentistry for the treatment or prevention of inflammatory processes such as mucositis or periimplantitis.

### BACKGROUND

The use of dental implants based on titanium pieces inserted in the mouth is nowadays a common practice in modern dentistry. Such implants typically afford a success rate of 89% in recovering the functional and aesthetic properties of the substituted dental piece. However, several complications may arise from the insertion of a dental implant in a patient. Among these complications, accumulation of microbial plaque in the surroundings of the inserted implant often leads to the development of infections in the area surrounding the implant. Such infections may stem from cross contamination during the surgery for inserting the implant in the mouth or from a deficient healing of the tissues. These infections may also arise in a delayed manner post-surgery as a result of accumulation of bacterial plaque on the surface of the implant. The infections are caused by bacterial colonization of the surrounding of the implant, in particular of the bone-implant interface. These complications often require removal of the implant. Periimplantitis is defined as the destructive process in the surrounding of an implant in process of bone integration, which may result in loss of bone support and incomplete or absent integration of the implant in bones. A complication of periimplantitis is thus the loss of the implant. This condition is a concern in the field, for it is observed in 20% of patients receiving an implant in a period of 1-5 years post-surgery.

Periimplantitis generally develops as a result of bacterial colonization followed by the formation of a biofilm by adhesion of bacteria to the surface of the implant and to already adhered bacteria. It has been observed in the art that different types of bacteria can be found on the surface of an implant after one hour of the surgical act and that a bacterial biofilm only needs two weeks to form. As a consequence, there is a need for intervening quickly in order to prevent the spread of the infection and eradicate the formed biofilm. Several bacteria have been identified as playing a role in the development of periimplantits. Such bacteria are for instance *Bacteroides, Campylobacter, Eubacterium, Fusobacterium, Treponema species, Aggregatibacter actinomycetemcomitans, Prevotella, intermedia, Porphyromonas gingivalis, Treponema denticola, Tannerella forsythia and, particularly, Staphylococcus aureus.* As a consequence, the search of surfaces with the ability to prevent adhesion of bacteria, formation of bacterial biofilms and bacterial colonization in the surrounding area of the implant is the object of intensive research.

Mucositis is an inflammatory process that arises in patients receiving a buccal implant wherein the tissues surrounding the implant are inflamed as a result of bacterial plaque accumulation. Mucositis usually precedes periimplantitis. In this regard, European patent EP1799186B1 discloses orally available pharmaceutical compositions comprising octenidine for the treatment or prevention of inflammatory diseases of the buccopharyngeal cavity.

Several anti-bacterial agents are known in the art for the prevention or treatment of periimplantitis. Those include salts, ions or nanoparticles of metals such as silver, gold, copper and zinc. Antibiotic compounds have also been extensively used as anti-bacterial agents. However, bacteria strains tend to develop some resistance to antibiotics, when exposed to them, thus leading to inefficient prevention or treatment strategies. Most commonly used antibiotics include gentamicin, cefalotinin, amoxicillin, metronidazole, tobramycin and vancomycin. These antibiotic agents are commonly introduced in biocompatible inorganic matrices such as calcium phosphate or hydroxyapatite or in polymeric coatings or in sol-gel matrices. In this regard, Radin and Ducheyne disclose in Biomaterials 28 (2007) 1721-1729 a sol-gel matrix prepared from tetraethylorthosilicate (TEOS) and incorporating vancomycin. The multi-layered disclosed matrix allows in particular to release the anti-bacterial agent in a controlled manner and is resorbable. In addition, a correlation is suggested between the degradation rate of the sol-gel matrix and the release rate of the antibiotic compound.

Further anti-bacterial agents known in the art include cationic compounds, such as quaternary ammonium compounds, antimicrobial peptides and natural molecules such as chitosan among others. However, quaternary ammonium compounds may lack selectivity over the bacteria and may present undesired cytotoxic effects. Salts of chlorhexidine and octenidine are also known cationic compounds useful as anti-bacterial agents. Octenidine salts, and in particular its dihydrochloride salt, are active against a broad range of bacteria and are stable in a broad range of conditions. Octenidine advantageously reacts with the polysaccharides found on the walls of microorganisms and inhibits cellular function, thereby preventing the growth of bacterial plaque. Octenidine further exhibits low cytotoxicity.

Coatings for releasing anti-bacterial agents and suitable for use in the treatment or prevention of periimplantitis are known in the art. In this regard, I. García-Arnáez, B. Palla, J. Suay, F. Romero-Gavilán, L. García-Fernández, M. Fernández, I. Goñi, and M. Gurruchaga disclose in European Polymer Journal, Volume 113, 2019, Pages 289-296 a hybrid organic-inorganic coating based on sol-gel materials with proven osteogenic capabilities and incorporating either

octenidine dihydrochloride or chlorhexidine diacetate as anti-bacterial agents. The disclosed systems are suitable for preventing the formation of biofilms and bacterial adhesion on the surface of the implant. The matrix is prepared by the sol-gel method employing as silicon-based reagents a mixture of tetraethylorthosilicate (TEOS) and methyl trimethoxysilane (MTMOS) in a molar ratio of 3:7. The formed sol-gel matrix is loaded with an anti-bacterial agent that is chlorhexidine diacetate or octenidine dihydrochloride in a weight ratio of 0-2%. According to this document, the release rate of the active ingredient is proportional to the degradation rate of the sol-gel matrix material. Also, the presence of TEOS in the sol-gel material is disclosed to promote the hydrophilic character of the material and, consequently, its hydrolytic degradation.

International patent application WO 2017/197510 A1 discloses a biocompatible composite material for the controlled release of octenidine. The biocompatible matrix comprises a mesoporous silicon oxide matrix which can be prepared as a coating. The active ingredient, i.e. octenidine, is predominantly released by diffusion through the pores of the mesoporous material, such that the matrix silicon oxide material is substantially not subject to degradation or hydrolysis. Tetraethylorthosilicate is disclosed as principal source of the silicon oxide mesoporous matrix. In addition, octenidine is employed as amphiphilic compound for the formation of micellar assemblies, at loads of between 30% and 40% in weight.

Spanish patent application ES201031831 discloses a process for the preparation of a sol-gel coating employing, among others, mixtures of tetraethylorthosilicate (TEOS) and methyltrimethoxysilane (MTMOS) as precursors, in molar ratios of 4:1, 2:1 and 4:3. The document is silent about the use of such sol-gel matrices for the release of anti-bacterial agents such as octenidine. This document also discloses that the curing of the sol material to form the gel can be carried out in severe and non-severe conditions. Curing in non-severe conditions tends to promote a fast degradation of the material, for the cured material presents a low degree of cross-linking.

From what is disclosed in the art, it derives that there is still a need for providing improved coatings suitable for the treatment or prevention of inflammatory processes such as mucositis or periimplantitis, in particular coatings suitable for: (i) forming a physical barrier between the implant surface and the physiological medium suitable for embedding bacteries and preventing adhesion of bacteria on the surface of the implant during the regeneration of soft tissue, (ii) releasing an anti-infective agent in a controlled manner, (iii) being cured in physiological conditions in a minimal period of time, (ivi) being used in living organisms, (v) degrading in physiological conditions and/or preventing the formation of fibrous capsule between the implant and the surrounding tissues

## SUMMARY OF THE INVENTION

After exhaustive research, the inventors have found that a coating composition comprising from 1% to 7.5% in weight of octenidine dihydrochloride embedded in a sol-gel material prepared from a mixture of silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1\text{-}C_4)$alkyl chain, such as tetraethylorthosilicate (TEOS), and a compound of formula $Si(R^5)(OR^6)(OR^7)(OR^8)$ wherein each of $R^5$, $R^6$, $R^7$ and $R^8$ is independently a $(C_1\text{-}C_4)$alkyl chain, such as methyltrimethoxysilane (MTMOS), in a molar ratio of from 80:20 to 50:50, possesses beneficial properties for its use in the prevention or treatment of inflammatory processes such as mucositis or periimplantitis.

A first beneficial property is that said coating composition can be cured under conditions existing in a patient's mouth, i.e. a temperature of about 37 °C and a humidity of about 100%, in a time of about 10 minutes, contrary to what occurs with prior art octenidine compositions wherein the compound of formula $Si(R^5)(OR^6)(OR^7)(OR^8)$ (e.g. MTMOS) is the predominant component. It is advantageous as the coating composition can be applied directly during or after surgery with minimal discomfort to the patient within a generally accepted timeframe for this kind of operations and with no need of external heating or activation to trigger the curing.

A second benefit for the coating composition, when used for coating implants or prosthesis, is that it advantageously allows forming a solid film on the implant surface, said film acting as a physical barrier between bacteria from the physiological medium and the surface of the implant. It is believed that the film is also suitable for embedding the bacteria adhered to the substrate surface, thus resulting in the elimination of bacterial population adhered to the surface of the substrate. In some embodiments, the physical barrier is robust enough to allow for regeneration of soft tissue around the implant, thus preventing efficiently the development of an infection-related inflammatory response around the implant. This is surprisingly even the case when the coating composition of the invention is deprived of anti-bacterial agents.

A third benefit for the coating composition of the invention is that it advantageously allows releasing high amounts of octenidine in a sustained manner during the month following the application of the coating. The release behaviour of the coating composition of the present invention is particularly ideal in the context of periimplantitis, as it is capable of preventing initial and long-term infection through the sustained release of octenidine, thus providing a longer period for implant integration protected against infection. This is unexpected as the above-described state of the art discloses coating compositions comprising 2% octenidine, and a compound of formula $Si(OR^1)(OR^2)(OR^3)(OR^4)$ (e.g. TEOS) in relatively low amounts, wherein practically all of the octenidine released in the month following the application of the coating is released within a period of about 7 days and is lower than the amount of octenidine comprised in the coating at the time of application. The prior art further teaches that an increase of the amount of TEOS in the silicon-based sol-gel

precursors leads to a faster degradation of the gel matrix and that the degradation of the matrix is directly related with the release of octenidine. In other words, the skilled person would have expected the octenidine in a coating composition according to the present invention to be released in an even more rapid manner compared to low-TEOS prior art coating compositions, and thus would have not considered such a high-TEOS coating composition suitable for the treatment of periimplantitis, wherein delayed infection is of concern.

The coating composition of the invention is also advantageously biocompatible and exhibits excellent bactericide properties. In particular, and unlike the coatings disclosed in the art, the coating composition of the invention advantageously and surprisingly does not lead to the formation of a fibrous capsule between the implant and the tissues surrounding the implant, thus promoting an efficient integration of the implant in bones. In addition, the process of preparation of said coating composition is advantageously short (inferior to 30 minutes) such that it can be carried out within the dentist consultation with no specific discomfort for the practitioner and the patient and during the visit of the patient. In addition, no specific equipment is required to carry out said process, such that a practitioner does not need additional equipment to prepare the coating composition of the invention.

Thus, in a first aspect, the invention relates to a process for the preparation of a coating composition comprising the steps of:

(i) providing a mixture comprising silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1\text{-}C_4)$alkylchain, preferably tetraethoxysilane, and a compound of formula $Si(R^5)(OR^6)(OR^7)(OR^8)$ wherein each of $R^5$, $R^6$, $R^7$ and $R^8$ is independently a $(C_1\text{-}C_4)$alkylchain, preferably methyltrimethoxysilane, in a molar ratio of from 80:20 to 50:50, the mixture further comprising octenidine in an amount of from 1 to 7.5 grams per each 100 grams of the silicon-based sol-gel precursors;
(ii) treating the mixture provided in step (i) with at least an effective amount of an aqueous acidic solution so as to form a sol material;
(iii) optionally, curing the sol material of step (ii) by heating said material at a temperature of about mouth temperature.

A second aspect of the invention relates to a composition obtainable by the process as defined in the first aspect of the invention.

The composition of the second aspect of the invention is particularly useful in the prevention or treatment of inflammatory processes such as mucositis or periimplantitis, preferably of periimplantitis.

A third aspect of the invention thus relates to a composition according to the second aspect of the invention for use in medicine.

A fourth aspect of the invention relates a composition according to the second aspect of the invention for use in the prevention or treatment of inflammatory processes such as mucositis or periimplantitis, preferably of periimplantitis.

As mentioned above, the composition of the second aspect of the invention may be prepared in two steps. A kit for the preparation of the product of the second aspect of the invention is also part of the invention, said kit comprising, in a first alternative, reagents for the preparation of the pre-cured material, and, in a second alternative the pre-cured material. Thus, a fifth aspect of the invention thus relates to a kit of parts for preparing a substrate coated with an anti-infective coating composition comprising:

- in a first part, a mixture as provided in step (i) of the process defined in the first aspect of the invention,
- in a second part, an aqueous acidic solution as employed in step (ii) of the process defined in the first aspect of the invention,
- in an optional third part means for mixing the content of the first and second parts, and, optionally, means for heating the resulting mixture, and
- in an optional fourth part, a substrate for receiving an optionally heated mixture of the content of said first and second parts, and, optionally, means for transferring said optionally heated mixture to the substrate;

or, alternatively,

- in a first part, a sol material obtainable by the process comprising steps (i) and (ii) as defined in the first aspect of the invention, and
- in a second part, a substrate for receiving said sol material and, optionally, means for transferring said sol material to said substrate.

A sixth aspect of the invention relates to a mixture as provided in step (i) of the process defined in the first aspect of the invention.

## BRIEF DESCRIPTION OF THE DRAWINGS

Fig. 1 shows the rate of hydrolytic degradation of materials M1-M5 from Example 1 as a measure of weight loss (expressed as a percentage) in function of time (expressed in days).
Fig. 2 shows the release profile of octenidine (expressed as the ratio of the accumulated amount of octenidine released at a certain point in time over the amount of octenidine present in the coating composition) as a function of time (expressed in days) for samples M2 -M5 as prepared in Example 1.
Fig. 3 shows the cell viability of human fibroblast cells (FBH, expressed as a percentage) in function of time ((a): 1 day, (b): 2 days. (c): 7 days)) put in contact with samples M1-M5 as prepared in Example 1 and measured according to MTT cell viability assay, ANOVA test, *$p<0.05$ with respect to control Ti disk (uncoated disk).
Fig. 4 shows the cell viability of human osteoblast cells (MG-63, expressed as a percentage) in function of time ((a): 1 day, (b): 2 days, (c): 7 days)) put in contact with samples M1-M5 as prepared in Example 1 and measured according to according to MTT cell viability assay, ANOVA test, *$p<0.05$ with respect to control Ti disk (uncoated disk).
Fig. 5 shows the cell proliferation of human fibroblast cells (FBH, expressed as a fluorescence intensity) in function of time ((a): 1 day, (b): 2 days. (c): 7 days)) put in contact with samples M1-M5 as prepared in Example 1 and measured according to ALAMAR BLUE cell proliferation assay.
Fig. 6 shows the cell proliferation of human osteoblast cells (MG-63, expressed as a fluorescence intensity) in function of time ((a): 1 day, (b): 2 days. (c): 7 days)) put in contact with samples M1-M5 as prepared in Example 1 and measured according to ALAMAR BLUE cell proliferation assay.
Fig. 7 shows the relative value of cell viability, expressed as a percentage, of a culture of Staphylococcus aureus CECT 86 put in contact with titanium disks coated with the material M1-M5 following the procedure of Example 1 for measuring bactericide effect.
Fig. 8 shows the release profile of octenidine (expressed as the ratio of the accumulated amount of octenidine released at a certain point in time over the amount of octenidine present in the coating composition) as a function of time (expressed in days) for comparative samples C1 -C4 and sample M3 as prepared in Example 1.
Fig. 9 shows the semi-qualitative assessment of the response of bone marrow to transplantation with (left) a control titanium implant and (right) a titanium implant coated with M3 over periods of time of 1, 2 or 4 weeks, in terms of (1) aplasia, (2) architectural loss, (3) aspect of zones of bone marrow not in contact with implant, and (4) fat ratio.
Fig. 10 shows the semi-qualitative assessment of the response of the periimplantar fibrous capsule to transplantation with (left) a control titanium implant and (right) a titanium implant coated with M3 over periods of time of 1, 2 or 4 weeks, at the following locations (1) bone marrow, (2) between the implant and the cortical, (3) between the implant and the trabecular bone, and (4) degree of densification.
Fig. 11 shows photographs of metal substrates coated with materials M1, M2 or M5 of example 1 (left column) and photographs of said metal substrate after carrying out the adhesion test of ISO2409 (cross cut assay, right column).
Fig. 12 shows photographs of steel substrates coated with materials C1, C2 or M6 of example 1 after treatment at 37 °C at 100% humidity for 10 minutes and after wiping out the top lateral side of the metal piece with a paper tissue.
Fig. 13 shows histological images of the area of cortical and trabecular bones in contact with a coated titanium implant for (top-left) implant coated with material M6 after a period of 2 weeks from implantation; (top-right) implant coated with material M6 after a period of 8 weeks from implantation at trabecular bone; (bottom-left) implant coated with a sol-gel material made from a mixture of 90% MTMOS and 10% TEOS (molar) after a period of 8 weeks from implantation at trabecular bone; (bottom-right) implant coated with a sol-gel material made from a mixture of 90% MTMOS and 10% TEOS (molar) after a period of 8 weeks from implantation near medullary cavity.
Fig. 14 shows a microscope picture of a periimplant mucosa area showing the areas of interest in the clinical studies carried out on beagle dogs: E: epithelium, TCI1: Infiltrated connective tissue in connective papillae, TCI2: Infiltrated connective tissue in areas remote from epithelium.
Fig. 15 shows a microscope picture of a periimplant mucosa sample taken at T0 in the clinical trials carried out on beagle dogs.
Fig. 16 shows a microscope picture of a periimplant mucosa sample taken at T3 in the clinical trials carried out on beagle dogs.
Fig. 17 shows a microscope picture of a periimplant mucosa sample taken at T6 in the clinical trials carried out on beagle dogs.
Fig. 18 shows a microscope picture of a periimplant mucosa sample taken at T7 in the clinical trials carried out on beagle dogs.
Fig. 19 shows a microscope picture of a periimplant mucosa sample taken at T10 in the clinical trials carried out on beagle dogs (treated and control groups).
Fig. 20 shows a microscope picture of a periimplant mucosa sample treated to detect substance P in an immunohistochemical assay carried out on sample of dogs from the control group (top) and from the treated group (bottom).
Fig. 21 shows microscope pictures of stained bone samples obtained from histological experiments of the bone of a beagle dog receiving an implant treated with mixture M3 (top) and a beagle dog receiving an untreated implant (bottom).
Fig. 22 shows pictures of proliferation of Streptococcus Gordonii bacteria after one day of incubation at 37 °C on the surface of grade-IV titanium disks coated with: a) nothing; b) mixture M1 of Example 1; c) mixture M3 of Example 1 and; d) a solution of octenidine.

## DETAILED DESCRIPTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the invention, any ranges given include both the lower and the upper end-points of the range. Ranges or values given, such as temperatures, times, molar ratio, volume ratio and the like, should be considered approximate when they are defined by the term "about" (i.e. with a 5% margin of variation around indicated point).

In the context of the invention, the term "alkyl" refers to an aliphatic saturated hydrocarbon chain that is linear or branched and having the number of carbon atoms defined in the claims and the description. Non-limiting examples of alkyl groups include, for instance, methyl, ethyl, *n*-propyl, *i*-propyl, *n*-butyl, *i*-butyl, *t*-butyl, *n*-pentyl, neo-pentyl, and hexyl.

In the context of the invention, the term "octenidine" refers both to octenidine and to one of its known salts, such as the dihydrochloride salt of octenidine. Octenidine is a compound of formula

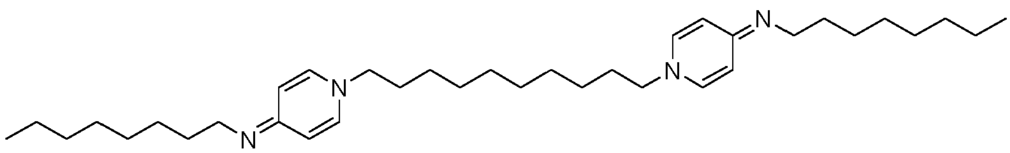

whereby the imine groups may be protonated. In the case of the dihydrochloride salt of octenidine, both imine groups are protonated. In a preferred embodiment, the octenidine is the dihydrochloride salt of octenidine.

In the context of the invention, the term "silicon-based sol-gel precursor" refers to organosilicon compounds known in the art and suitable for the formation of sol-gel materials of the polysiloxane type. In particular embodiments of the present invention, the term "silicon based sol-gel precursor" refers to a compound of formula $Si((O)_nR^a)(OR^b)(OR^c)(OR^d)$ wherein n is 0 or 1 and each of $R^a$, $R^b$, $R^c$ and $R^d$ is independently selected from the group consisting of $(C_1\text{-}C_{12})$alkyl, $(C_2\text{-}C_{12})$alkenyl and $(C_6\text{-}C_{20})$aryl wherein the alkyl and alkenyl chains are optionally substituted with one or more groups selected from halogen amino, and groups suitable for cross-linking, such as glycidyl, (meth)acrylate or a thiol group. Known examples of silicon-based sol-gel precursors include tetramethoxysilane, tetraethoxysilane (TEOS), methyl trimethoxysilane (MTMOS), vinyl trimethoxysilane, vinyl triethoxysilane, phenyl triethoxysilane, 3-aminopropyltriethoxysilane, (3-glycidyloxy)propyltrimethoxysilane (GPTMS), and 3-(trimethoxysilyl) propyl methacrylate. Particularly, useful are TEOS, MTMOS and GMTPS:

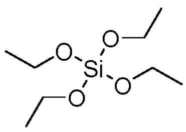

TEOS

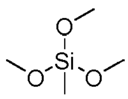

MTMOS

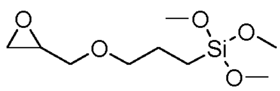

GPTMS

In the context of the invention, the term "effective amount", when used in the context of formation of a sol material, refers to an amount of acid inducing the condensation of the silicon-based sol-gel precursors and the formation of sol materials. In particular embodiments, such effective amount is given when the pH of the aqueous acidic solution is of between 1 and 2.

In the context of the present invention, the term "prevention or treatment", as used herein, comprises any type of therapy, which aims at terminating, preventing, ameliorating and/or reducing the susceptibility to a clinical condition as described herein, e.g. bacterial infection. Thus, "prevention or treatment," "preventing or treating," and the like, as used herein, refer to obtaining a desired pharmacologic and/or physiologic effect, covering any treatment of a pathological condition or disorder in a mammal, including a human. The effect may be prophylactic in terms of completely or partially

preventing a disorder or symptom thereof and/or may be therapeutic in terms of a partial or complete cure for a disorder and/or adverse effect attributable to the disorder. That is, "prevention or treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter. Those in need of treatment include those already with the condition or disorder as well as those prone to have the condition or disorder or those in which the condition or disorder is to be prevented.

In the context of the invention, the term "sol material" refers to a colloidal solution or suspension of polysiloxanes. In contrast, a "gel material" refers to an integrated network comprising polysiloxanes and/or discrete particles of polysiloxanes that are reticulated or cross-linked. Typically, during a sol-gel process, a silicon-based sol gel precursor is converted into a sol material, which in turn serves as precursor of the gel material.

In the context of the invention, the term "mouth temperature" refers to the average temperature of the mouth of the human body, that is, a temperature comprised between 30 °C and 40 °C, preferably a temperature of about 37 °C.

As mentioned above, a first aspect of the invention relates to a process for the preparation of a coating composition comprising the steps of:

(i) providing a mixture comprising a compound of formula $Si(OR^1)(OR^2)(OR^3)(OR^4)$ wherein each of $R^1$, $R^2$, $R^3$ and $R^4$ is independently a $(C_1\text{-}C_4)$alkyl chain and a compound of formula $Si(R^5)(OR^6)(OR^7)(OR^8)$ wherein each of $R^5$, $R^6$, $R^7$ and $R^8$ is independently a $(C_1\text{-}C_4)$alkyl chain, in a molar ratio of from 80:20 to 50:50, the mixture further comprising octenidine in an amount of from 1 to 7.5 grams per each 100 grams of the silicon-based sol-gel precursors;
(ii) treating the mixture provided in step (i) with at least an effective amount of an aqueous acidic solution so as to form a sol material;
(iii) optionally, curing the sol material of step (ii) by heating said material at a temperature of about mouth temperature.

In a particular embodiment of the first aspect of the invention, the compound of formula $Si(OR^1)(OR^2)(OR^3)(OR^4)$ comprised in the silicon-based sol-gel precursors is tetraethylorthosilicate (TEOS). The terms "tetraethylorthosilicate", "tetraethoxysilane" and "TEOS" all herein refer to the same compound of formula $Si(OEt)_4$ and are used interchangeably.

In a particular embodiment of the first aspect of the invention, the compound of formula $Si(R^5)(OR^6)(OR^7)(OR^8)$ comprised in the silicon-based sol-gel precursors is methyltrimethoxysilane (MTMOS). The terms "methyltrimethoxysilane" and "MTMOS" all herein refer to the same compound of formula $MeSi(OMe)_3$ and are used interchangeably.

In a particular embodiment of the first aspect of the invention, the molar ratio of the compound of formula $Si(OR^1)(OR^2)(OR^3)(OR^4)$ to the compound of formula $Si(R^5)(OR^6)(OR^7)(OR^8)$ comprised in the silicon-based sol-gel precursors is of from 70:30 to 50:50. Preferably, it is from 65:35 to 55:45. Even more preferably, it is 60:40.

In a particular embodiment of the first aspect of the invention, the silicon-based sol-gel precursors of the mixture of step (i) comprise tetraethoxysilane and methyltrimethoxysilane in a molar ratio of from 65:35 to 55:45. Preferably, the silicon-based sol-gel precursors of the mixture of step (i) comprise tetraethoxysilane and methyltrimethoxysilane in a molar ratio of 60:40.

In a particular embodiment, the silicon-based sol-gel precursors of the mixture of step (i) comprise the compound of formula $Si(OR^1)(OR^2)(OR^3)(OR^4)$ and the compound of formula $Si(R^5)(OR^6)(OR^7)(OR^8)$ in an amount of at least 50%, preferably at least 80%, more preferably at least 95%, even more preferably at least 99%, by weight with respect to the total weight of the silicon-based sol-gel precursors.

In a particular embodiment, the silicon-based sol-gel precursors of the mixture of step (i) comprise the tetraethoxysilane and methyltrimethoxysilane in an amount of at least 50%, preferably at least 80%, more preferably at least 95%, even more preferably at least 99%, by weight with respect to the total weight of the silicon-based sol-gel precursors.

In a particular embodiment, the silicon-based sol-gel precursors of the mixture of step (i) consist of the compound of formula $Si(OR^1)(OR^2)(OR^3)(OR^4)$ and the compound of formula $Si(R^5)(OR^6)(OR^7)(OR^8)$, preferably tetraethoxysilane and methyltrimethoxysilane. The molar ratio of tetraethoxysilane to methyltrimethoxysilane may be any of those mentioned above.

In an embodiment, in any embodiment described herein, the silicon-based sol-gel precursors of the mixture of step (i) do not comprise glycidoxypropyltrimethoxysilane.

In a more particular embodiment of the first aspect of the invention, the silicon-based sol-gel precursors consists of a mixture of tetraethoxysilane and methyltrimethoxysilane in a molar ratio of 3:2.

In a particular embodiment of the first aspect of the invention, the mixture of step (i) consists essentially of the silicon-based sol-gel precursors as defined above in any particular or preferred embodiment and octenidine in an amount of from 1 to 7.5 grams per each 100 grams of the silicon-based sol-gel precursors.

In the context of the invention, the term "consists essentially of" means that specific further components can be present in the mixture of step (i), namely those not materially affecting the essential characteristics of the mixture, in particular its ability to form sol-gel materials of the polysiloxane type with antiseptic activity.

As defined in the first aspect of the invention, the mixture of step (i) comprises octenidine in an amount of from 1 to 7.5 grams per each 100 grams of the silicon-based sol-gel precursors. Preferably, the mixture of step (i) comprises octenidine in an amount of from 1 to 6 grams; more preferably from 1.5 to 5 grams; even more preferably from 2 to 4 grams; per each 100 grams of the silicon-based sol-gel precursors. Even more preferably, the mixture of step (i) comprises octenidine in an amount of from 2 to 3.5 grams; more particularly of from 2 to 3 grams; and even more particularly of about 2 grams per each 100 grams of the silicon-based sol-gel precursors.

In particular embodiments of the first aspect of the invention, the mixture of step (i) comprises octenidine in an amount of 1, 2, 5 or 7.5 grams per each 100 grams of the silicon-based sol-gel precursors.

In another particular embodiment of the first aspect of the invention, the mixture of step (i) comprises octenidine in an amount of 2 grams per each 100 grams of the silicon-based sol-gel precursors.

In a preferred embodiment, the mixture of step (i) comprises silicon-based sol-gel precursors comprising at least 80% by weight of a mixture of the compound of formula $Si(OR^1)(OR^2)(OR^3)(OR^4)$ and the compound of formula $Si(R^5)(OR^6)(OR^7)(OR^8)$, preferably of a mixture of tetraethoxysilane and methyltrimethoxysilane, in a molar ratio of 65:35 to 55:45 and octenidine in an amount of from 2 to 4 grams per each 100 grams of the silicon-based sol-gel precursors.

In a preferred very particular embodiment, the mixture of step (i) comprises silicon-based sol-gel precursors consisting of a mixture of tetraethoxysilane and methyltrimethoxysilane in a molar ratio of 60:40 and octenidine in an amount of 2 grams per each 100 grams of the silicon-based sol-gel precursors.

In a preferred embodiment of the first aspect of the invention, the mixture of step (i) further comprises a solvent. Suitable solvents are those known in the art for the formation of polysiloxanes by the sol-gel method. Typically suitable solvents are polar protic organic solvents such as alcohols. Such alcohols include, among others, methanol, ethanol, propanol, iso-propanol, butanol and tert-butanol. A preferred solvent is iso-propanol.

When the mixture of step (i) comprises a solvent, said solvent is preferably in an amount such that the ratio of the volume of the solvent to the volume of the silicon-based sol-gel precursors is from 1:2 to 2:1. More preferably, the solvent is in an amount such that the ratio of the volume of the solvent to the volume of the silicon-based sol-gel precursors is about 1:1.

Thus, in a more preferred embodiment of the first aspect of the invention, the mixture of step (i) comprises silicon-based sol-gel precursors consisting of a mixture of tetraethoxysilane and methyltrimethoxysilane in a molar ratio of 3:2 and octenidine in an amount of 1, 2, 5 or 7.5 grams per each 100 grams of the silicon-based sol-gel precursors and further comprises isopropanol, wherein the isopropanol is preferably in an amount such that the ratio of the volume of the solvent to the volume of the silicon-based sol-gel precursors is about 1:1.

The process of the first aspect of the invention relates to the formation of a cross-linked polysiloxane network whereby octenidine is embedded. The second step of the process of the first aspect of the invention relates to the formation of said network which takes place in acidic aqueous conditions. The use of such conditions allows favouring the polycondensation of silicon-based sol-gel precursors over their hydrolysis, and thus favours the formation of cross-linked polysiloxane compounds with linear sections.

Thus, step (ii) of the first aspect of the invention relates to treating the mixture of step (i) with at least an effective amount of an aqueous acidic solution so as to form a sol material. Said aqueous acidic solution may be an acidic solution of any of hydrochloric acid, acetic acid, citric acid, sulfuric acid, phosphoric acid, nitric acid, boric acid and mixtures thereof. Preferably, step (ii) of the first aspect of the invention relates to treating the mixture of step (i) with at least an effective amount of an aqueous solution of nitric acid.

Said effective amount of acidic aqueous solution is preferably a sub-stoichiometric or stoichiometric amount of acid. The acid is useful for accelerating the hydrolysis of the alkoxyy groups comprised in the silicon-based gel precursors.

In particular embodiments of the first aspect of the invention, the aqueous acidic solution of step (ii) has a pH of between 1 and 2. The skilled person will devise the volume of acidic solution to add to the mixture of step (i) on the basis of the pH of the solution and the effective amount of acid to be added to the mixture of step (i).

In other particular embodiments of the first aspect of the invention, the aqueous acidic solution of step (ii), preferably a solution of nitric acid, possesses an acid concentration of from 0.01 N to 0.5 N; preferably 0.01 N to 0.1 N. More preferably, it is a solution of nitric acid in a concentration of 0.1 N.

In other particular embodiments of the first aspect of the invention, step (ii) is carried out at a temperature of between 25 °C and 100 °C. Preferably, step (ii) is carried out at a temperature of between 50°C and 100°C, more preferably of about 70 °C. When step (ii) is carried out at a temperature of 70 °C, it advantageously allows forming a sol material in a short period of time, i.e. of about 30 minutes. This advantageously allows preparing the coating composition during the surgery or the visit of the patient to the consultation.

In more particular embodiments of the first aspect of the invention, the mixture of step (i) comprises silicon-based sol-gel precursors consisting of a mixture of tetraethoxysilane and methyltrimethoxysilane in a molar ratio of 3:2 and

octenidine in an amount of 1, 2, 5 or 7.5 grams per each 100 grams of the silicon-based sol-gel precursors, and step (ii) comprises treating the mixture of step (i) with a solution of nitric acid in a concentration of from 0.01 N to 0.5 N at a temperature of between 50 °C and 100 °C; preferably the solution of nitric acid is in a concentration 0.01 N to 0.1 N and step (ii) is carried out at 70 °C. In said embodiment, it is further preferred that step (ii) is carried out for a period of time of about 30 minutes.

The combination of steps (i) and (ii) allows preparing a sol material which, following curing, is able to form a physical barrier between the implant surface and the physiological medium suitable for embedding bacteries and preventing adhesion of bacteria on the surface of the implant during the regeneration of soft tissue. Additionally, said sol material is able to release octenidine in a sustained manner over a prolonged period of time. This composition is thus particularly useful as anti-infective coating of implants, such as dental or bone implant. It is thus further contemplated that the process of the first aspect of the invention comprises the partial or complete coating of a substrate for implanting or of an implanted substrate, such as a dental or bone implant, with the sol material. In a particular embodiment, the substrate is a substrate for implanting. In another particular embodiment, the substrate is an implanted substrate. In another particular embodiment, the substrate is a material for use as a closure cap in dentistry. Materials suitable for use as closure caps in dentistry are well known in the art and will become apparent to the skilled person upon reduction to practice of the invention. Materials suitable for use as closure caps in dentistry include, for instance, collagen, titanium, stainless steel, polyethylene, polypropylene, medical-grade silicone, polyglycolic acid, polylactic acid, polydioxanone and caprolactone. These materials, and in particular collagen, may be used to form sponges, membranes, plugs and matrices which are useful in dentistry. The transfer of the sol product to the substrate in the coating step may be carried in a first particular alternative after step (ii). Since the product of step (ii) is a sol material, it has a sufficient viscosity to allow for coating the substrate. Prior to the transfer to the substrate, the sol material prepared in step (ii) may further be aged, preferably under ambient conditions of temperature and moisture, which allows for increasing the viscosity of the sol material and allows a more precise application of the coating composition on the surface of the substrate or implant. No specific additives for improving the adhesion of the coating composition to the substrate are required to carry out said transfer, although they may be added to the sol material in order to improve the adhesion of the sol material to the surface of the substrate. It is however preferred that such additives are not employed.

The transfer of the sol product to the substrate may be carried in a second particular alternative after step (iii), when step (iii) is carried out.

In preferred embodiments, the substrate comprises a surface, preferably a metal or polymer surface, comprising a metal selected from magnesium and magnesium alloys, titanium and titanium alloys or a polymer such as PEEK. Preferably, said metal is of surgical grade quality. More preferably, the metal is titanium or an alloy thereof.

In further particular embodiments, the substrate is a material for use as a closure cap in dentistry. Materials suitable for use as closure caps in dentistry are well known in the art and will become apparent to the skilled person upon reduction to practice of the invention. Materials suitable for use as closure caps in dentistry include, for instance, collagen, titanium, stainless steel, polyethylene, polypropylene, medical-grade silicone, polyglycolic acid, polylactic acid, polydioxanone and caprolactone. These materials, and in particular collagen, may be used to form sponges, membranes, plugs and matrices which are useful in dentistry.

The step of transferring the sol product of step (ii) or the gel product of step (iii) to the substrate is in an embodiment a non-therapeutic and/or non-surgical step, in the sense that it is not practiced on the human or animal body.

Alternatively, the step of transferring said product of step (ii) or of step (iii) to the substrate is carried out to achieve coating of an implant present in the mouth of a human or animal. In such cases, the invention refers to said product for use in medicine as described herein below.

In further particular embodiments, the process of the first aspect of the invention further comprises step (iii) of curing the sol material of step (ii) by heating said material at a temperature of about mouth temperature. The inventors have found that the sol material formed in step (ii) is able to form a gel in a minimal amount of time of about 10 minutes when heated at 37 °C. This is advantageous as it allows minimal discomfort to the patient. Step (iii) of the first aspect of the invention also advantageously may be performed in the presence of moisture. This advantageously allows for carrying out step (iii) in mouth conditions.

As defined above, a second aspect of the invention relates to a composition obtainable by the process as defined in the first aspect of the invention.

The second aspect of the invention thus relates to a composition obtainable by the process as defined in any of the particular and preferred embodiments of the first aspect of the invention defined above.

In a first alternative, the second aspect of the invention may thus relate to a sol material. This is particularly the case when step (iii) of the particular and preferred embodiments of the process of the first aspect of the invention defined above is not carried out.

Thus, the first alternative of the second aspect of the invention relates in a preferred embodiment to a sol material obtainable by the process which comprises the steps of:

(i) providing a mixture comprising silicon-based sol-gel precursors silicon-based sol-gel precursors comprising at least 80% by weight of a mixture of the compound of formula $Si(OR^1)(OR^2)(OR^3)(OR^4)$ and the compound of formula $Si(R^5)(OR^6)(OR^7)(OR^8)$, preferably of a mixture of tetraethoxysilane and methyltrimethoxysilane, in a molar ratio of 65:35 to 55:45 and octenidine in an amount of from 2 to 4 grams per each 100 grams of the silicon-based sol-gel precursors, and
(ii) treating the mixture of step (i) with a solution of acid in a concentration of from 0.01 N to 0.1 N and at a temperature of between 50 °C and 100 °C.

The first alternative of the second aspect of the invention relates in a preferred more particular embodiment to a sol material obtainable by the process which comprises the steps of:

(i) providing a mixture comprising silicon-based sol-gel precursors consisting of a mixture of tetraethoxysilane and methyltrimethoxysilane in a molar ratio of 3:2 and further comprising octenidine in an amount of 1, 2, 5 or 7.5 grams per each 100 grams of the silicon-based sol-gel precursors, and
(ii) treating the mixture of step (i) with a solution of nitric acid in a concentration of from 0.01 N to 0.1 N at a temperature of 70 °C.

In a second alternative, the second aspect of the invention may thus relate to a gel material. This is particularly the case when step (iii) of the particular and preferred embodiments of the process of the first aspect of the invention defined above is carried out.
Thus, the second alternative of the second aspect of the invention particularly relates in a preferred embodiment to a gel material obtainable by the process which comprises the steps of:

(i) providing a mixture comprising silicon-based sol-gel precursors comprising at least 80% by weight of a mixture of the compound of formula $Si(OR^1)(OR^2)(OR^3)(OR^4)$ and the compound of formula $Si(R^5)(OR^6)(OR^7)(OR^8)$, preferably of a mixture of tetraethoxysilane and methyltrimethoxysilane, in a molar ratio of 65:35 to 55:45 and octenidine in an amount of from 2 to 4 grams per each 100 grams of the silicon-based sol-gel precursors, and
(ii) treating the mixture of step (i) with a solution of acid in a concentration of from 0.01 N to 0.1 N and at a temperature of between 50 °C and 100 °C, and
(iii) curing the sol material obtained in step (ii) by heating said material at a temperature of between 30 °C and 40 °C.

Thus, the second alternative of the second aspect of the invention particularly relates in a preferred embodiment to a sol material obtainable by the process which comprises the steps of:

(i) providing a mixture comprising silicon-based sol-gel precursors consisting of a mixture of tetraethoxysilane and methyltrimethoxysilane in a molar ratio of 3:2 and further comprising octenidine in an amount of 1, 2, 5 or 7.5 grams per each 100 grams of the silicon-based sol-gel precursors,
(ii) treating the mixture of step (i) with a solution of nitric acid in a concentration of from 0.01 N to 0.1 N at a temperature of 70 °C, and
(iii) curing the sol material of step (ii) by heating said material at a temperature of about about 37 °C.

In a third alternative, when the process of the first aspect of the invention comprises the transfer of a sol or a gel material to a substrate, such as a dental, bone implant or a material for a closure cap, the product of the second aspect of the invention relates to a substrate, such as a dental, bone implant or a material for a closure cap, coated with a sol or a gel as defined in the first and second alternatives of the second aspect of the invention detailed above.
In particular embodiments, said substrate is suitable for receiving an anti-infective coating.
As mentioned above, said substrate preferably comprises a surface comprising a metal such as magnesium and magnesium alloys, titanium or titanium alloys, or a polymer such as PEEK, said metal being preferably of surgical grade quality. More preferably, said metal is titanium or an alloy thereof.
In further particular embodiments, the substrate is a material for use as a closure cap in dentistry. Materials suitable for use as closure caps in dentistry are well known in the art and will become apparent to the skilled person upon reduction to practice of the invention. Materials suitable for use as closure caps in dentistry include, for instance, collagen, titanium, stainless steel, polyethylene, polypropylene, medical-grade silicone, polyglycolic acid, polylactic acid, polydioxanone and caprolactone. These materials, and in particular collagen, may be used to form sponges, membranes, plugs and matrices which are useful in dentistry.
Thus, a particular embodiment of the third alternative of the second aspect of the invention relates to a dental, bone implant cap comprising a surface comprising titanium, said surface being coated with a sol or a gel as defined in the first and second alternatives of the second aspect of the invention detailed above.

Another particular embodiment of the third alternative of the second aspect of the invention relates to a closure cap comprising a surface of a material suitable for a closure cap as defined above, said surface being coated with a sol or a gel as defined in the first and second alternatives of the second aspect of the invention detailed above.

As defined above, a third aspect of the invention relates to a product as defined in the second aspect of the invention for use in medicine.

Particular embodiments of the third aspect of the invention relate to products as defined in any of the particular or preferred embodiments of the first (sol), second (gel) and third (coated substrate) alternatives of the second aspect of the invention for use in medicine.

As illustrated by the Examples below, the product of the second aspect of the invention is particularly useful in the prevention or treatment of inflammatory processes such as mucositis or periimplantitis, preferably of periimplantitis.

A fourth aspect of the invention relates to a product as defined in the second aspect of the invention for use in the prevention or treatment of inflammatory processes such as mucositis or periimplantitis, preferably of periimplantitis. Particular embodiments of the fourth aspect of the invention relate to products as defined in any of the particular or preferred embodiments of the first (sol), second (gel) and third (coated substrate) alternatives of the second aspect of the invention for use in the prevention or treatment of inflammatory processes such as mucositis or periimplantitis, preferably of periimplantitis.

In preferred embodiments of the invention, the inflammatory process susceptible of being treated or prevented by the compositions of the invention is one caused by one or more bacteria. Preferably, said one or more bacteria is one of the *Staphylococcus genus; particularly, Staphylococcus aureus, or is one of the Streptococcus genus; particularly, Streptococcus Gordonii,* or is selected from the group consisting of *Bacteroides, Campylobacter, Eubacterium, Fusobacterium, Treponema species, Aggregatibacter actinomycetemcomitans, Prevotella, intermedia, Porphyromonas gingivalis, Treponema denticola,* and *Tannerella forsythia.* In more particular embodiments, said one or more bacteria is *Staphylococcus aureus.* In other more particular embodiments, said one or more bacteria is *Streptococcus Gordonii.*

In further embodiments, the invention relates to the use of a composition according to the second or sixth aspect of the invention as defined in any of the embodiments provided herein in the manufacture of a coating or a coated substrate for the prevention or treatment of inflammatory processes such as mucositis or periimplantitis, preferably of periimplantitis.

In further embodiments, the invention relates to the composition according to the second aspect of the invention, as defined in any of the embodiments provided herein, for use in a method of treatment or prevention of inflammatory processes such as mucositis or periimplantitis, preferably of periimplantitis which comprises the step of coating an implant or prosthesis or material for closure caps with a therapeutically or prophylactically effective amount of the composition according to the second aspect of the invention, as defined in any of the embodiments provided herein.

A kit for the preparation of the products of the second aspect of the invention is also part of the invention and constitutes the fifth aspect of the invention.

The fifth aspect of the invention thus relates to a kit of parts for preparing a substrate coated with an anti-infective coating comprising:

- in a first part, a mixture as provided in step (i) of the process defined in the first aspect of the invention,
- in a second part, an aqueous acidic solution as employed in step (ii) of the process defined in the first aspect of the invention,
- in an optional third part means for mixing the content of the first and second parts, and, optionally, means for heating the resulting mixture, and
- in an optional fourth part, a substrate for receiving an optionally heated mixture of the content of said first and second parts and, optionally, means for transferring said optionally heated mixture to the substrate;

or, alternatively,

- in a first part, a sol material obtainable by the process comprising steps (i) and (ii) as defined in the first aspect of the invention, and
- in a second part, a substrate for receiving said sol material and, optionally, means for transferring said sol material to said substrate. In a particular embodiment, the first part of the first alternative of the kit of the fifth aspect of the invention relates to a mixture as provided in step (i) of the process as defined in any of the particular and preferred embodiments of the first aspect of the invention.

Thus, in more particular embodiments, the first part of the first alternative of the kit of the fifth aspect of the invention relates to a mixture comprising silicon-based sol-gel precursors consisting of a mixture of tetraethoxysilane and methyltrimethoxysilane in a molar ratio of 60:40 and further comprising octenidine in an amount of 1, 2, 5 or 7.5 grams per each 100 grams of the silicon-based sol-gel precursors. Said mixture further comprises preferably a solvent as defined above for the first aspect of the invention, said solvent being preferably iso-propanol.

In a particular embodiment, the second part of the first alternative of the kit of the fifth aspect of the invention relates to an acidic aqueous solution as defined in step (ii) of the process as defined in any of the particular and preferred embodiments of the first aspect of the invention.

Thus, in more particular embodiments, the second part of the first alternative of the kit of the fifth aspect of the invention relates to an aqueous solution of nitric acid in a concentration of from 0.01 N to 0.1 N. Preferably, it relates to an aqueous solution of nitric acid in a concentration of 0.1 N.

In a particular embodiment, the optional third part of the first alternative of the kit of the fifth aspect of the invention relates to means known in the art suitable for mixing the content of two separate parts comprising the compositions to be mixed. Said third part may further comprise heating means suitable for heating the resulting mixture at a temperature of between 50 °C and 100 °C. As will be obvious to the skilled person, the same heating means may be used for the curing of the sol material so as to form a gel. As defined above, said curing may preferably be carried out after the transfer of the sol material to the surface of a substrate, such as a dental or bone implant or a material for closure caps.

In a particular embodiment, the optional fourth part of the first alternative of the kit of the fifth aspect of the invention relates to a substrate as defined in any of the particular and preferred embodiments of the first aspect of the invention.

In a particular embodiment, the optional fourth part of the first alternative of the kit of the fifth aspect of the invention relates to a substrate comprising a surface, preferably a metal or a polymer surface, comprising a metal such as magnesium and magnesium alloys, titanium or titanium alloys that is preferably a metal of surgical grade quality, or a polymer such as PEEK. In more particular embodiments, said optional fourth part of the first alternative of the kit of the fifth aspect of the invention relates to a dental or bone implant, said implant comprising preferably a surface comprising titanium or an alloy thereof. In further more particular embodiments, said optional fourth part of the first alternative of the kit of the fifth aspect of the invention relates to a closure cap for dentistry. Materials suitable for use as closure caps in dentistry are well known in the art and will become apparent to the skilled person upon reduction to practice of the invention. Materials suitable for use as closure caps in dentistry include, for instance, collagen, titanium, stainless steel, polyethylene, polypropylene, medical-grade silicone, polyglycolic acid, polylactic acid, polydioxanone and caprolactone. These materials, and in particular collagen, may be used to form sponges, membranes, plugs and matrices which are useful in dentistry.

In another particular embodiment, the first part of the second alternative of the kit of the fifth aspect of the invention relates to a sol material obtainable by the process consisting of steps (i) and (ii) as defined in any of the particular and preferred embodiments of the first aspect of the invention or as defined in any of the particular and preferred embodiments first alternative of the second aspect of the invention.

In a particular embodiment, the second part of the second alternative of the kit of the fifth aspect of the invention relates to a substrate comprising a surface a metal such as magnesium and magnesium alloys, titanium or titanium alloys, or a polymer such as PEEK, said metal being preferably of surgical grade quality and being more preferably titanium or an alloy thereof. In more particular embodiments, said optional fourth part of the first alternative of the kit of the fifth aspect of the invention relates to a dental or bone implant that preferably comprises a surface comprising titanium that is preferably titanium of surgical grade quality.

In further more particular embodiments, second part of the second alternative of the kit of the fifth aspect of the invention relates to a substrate comprising a surface of a material suitable for use as closure caps in dentistry, such as collagen, titanium, stainless steel, polyethylene, polypropylene, medical-grade silicone, polyglycolic acid, polylactic acid, polydioxanone and caprolactone. These materials, and in particular collagen, may be used to form sponges, membranes, plugs and matrices which are useful in dentistry.

Essential components of the kit allowing for the preparation of a sol or gel material are the first and second parts of the first alternative of the kit of the fifth aspect of the invention.

Essential components of the kit allowing for the preparation of a substrate coated with an anti-infective coating composition are the first, second and fourth parts of the first alternative or the first and second parts of the second alternative of the kit of the fifth aspect of the invention.

The mixture of precursors reagent used in step (i) of the process of the first aspect of the invention is also part of the invention. Thus, the sixth aspect of the invention relates to a mixture as provided in step (i) of the process defined in any of the particular and preferred embodiments of the first aspect of the invention defined above.

In a particular embodiment of the sixth aspect of the invention, the compound of formula $Si(OR^1)(OR^2)(OR^3)(OR^4)$ comprised in the silicon-based sol-gel precursors is tetraethylorthosilicate (TEOS).

In a particular embodiment of the sixth aspect of the invention, the compound of formula $Si(R^5)(OR^6)(OR^7)(OR^8)$ comprised in the silicon-based sol-gel precursors is methyltrimethoxysilane (MTMOS).

In a particular embodiment of the sixth aspect of the invention, the molar ratio of the compound of formula $Si(OR^1)(OR^2)(OR^3)(OR^4)$ to the compound of formula $Si(R^5)(OR^6)(OR^7)(OR^8)$ comprised in the silicon-based sol-gel precursors is of from 70:30 to 50:50. Preferably, it is from 65:35 to 55:45. Even more preferably, it is 60:40.

In a particular embodiment of the sixth aspect of the invention, the silicon-based sol-gel precursors of the

composition of the sixth aspect of the invention comprise tetraethoxysilane and methyltrimethoxysilane in a molar ratio of from 65:35 to 55:45. Preferably, the silicon-based sol-gel precursors of the composition of the sixth aspect of the invention comprise tetraethoxysilane and methyltrimethoxysilane in a molar ratio of 60:40.

In a particular embodiment, the silicon-based sol-gel precursors of the composition of the sixth aspect of the invention comprise the compound of formula $Si(OR^1)(OR^2)(OR^3)(OR^4)$ and the compound of formula $Si(R^5)(OR^6)(OR^7)(OR^8)$ in an amount of at least 50%, preferably at least 80%, more preferably at least 95%, even more preferably at least 99%, by weight with respect to the total weight of the silicon-based sol-gel precursors.

In a particular embodiment of the sixth aspect of the invention, the silicon-based sol-gel precursors of the composition of the sixth aspect of the invention comprise tetraethoxysilane and methyltrimethoxysilane in an amount of at least 50%, preferably at least 80%, more preferably at least 95%, even more preferably at least 99%, by weight with respect to the total weight of the silicon-based sol-gel precursors.

In a particular embodiment of the sixth aspect of the invention, the silicon-based sol-gel precursors of the composition of the sixth aspect of the invention consist of the compound of formula $Si(OR^1)(OR^2)(OR^3)(OR^4)$ and the compound of formula $Si(R^5)(OR^6)(OR^7)(OR^8)$, preferably tetraethoxysilane and methyltrimethoxysilane. The molar ratio of tetraethoxysilane to methyltrimethoxysilane may be any of those mentioned above.

In a particular embodiment of the sixth aspect of the invention, the silicon-based sol-gel precursors of the composition of the sixth aspect of the invention do not comprise glycidoxypropyltrimethoxysilane.

In a more particular embodiment of the sixth aspect of the invention, the silicon-based sol-gel precursors consist of a mixture of tetraethoxysilane and methyltrimethoxysilane in a molar ratio of 3:2.

In a particular embodiment of the sixth aspect of the invention, the composition of the sixth aspect of the invention consists essentially of the silicon-based sol-gel precursors as defined above in any particular or preferred embodiment and octenidine in an amount of from 1 to 7.5 grams per each 100 grams of the silicon-based sol-gel precursors.

As defined in the sixth aspect of the invention, the composition of the sixth aspect of the invention comprises octenidine in an amount of from 1 to 7.5 grams per each 100 grams of the silicon-based sol-gel precursors. Preferably, the composition of the sixth aspect of the invention comprises octenidine in an amount of from 1 to 6 grams; more preferably from 1.5 to 5 grams; even more preferably from 2 to 4 grams; per each 100 grams of the silicon-based sol-gel precursors.

In particular embodiments of the sixth aspect of the invention, the composition of the sixth aspect of the invention comprises octenidine in an amount of 1, 2, 5 or 7.5 grams per each 100 grams of the silicon-based sol-gel precursors.

In another particular embodiment of the sixth aspect of the invention, the composition of the sixth aspect of the invention comprises octenidine in an amount of 2 grams per each 100 grams of the silicon-based sol-gel precursors.

In a preferred embodiment of the sixth aspect of the invention, the composition of the sixth aspect of the invention comprises silicon-based sol-gel precursors comprising at least 80% by weight of a mixture of the compound of formula $Si(OR^1)(OR^2)(OR^3)(OR^4))$ and the compound of formula $Si(R^5)(OR^6)(OR^7)(OR^8)$, preferably of a mixture of tetraethoxysilane and methyltrimethoxysilane, in a molar ratio of 65:35 to 55:45 and octenidine in an amount of from 2 to 4 grams per each 100 grams of the silicon-based sol-gel precursors.

In a preferred very particular embodiment, the composition of the sixth aspect of the invention comprises silicon-based sol-gel precursors consisting of a mixture of tetraethoxysilane and methyltrimethoxysilane in a molar ratio of 60:40 and octenidine in an amount of 2 grams per each 100 grams of the silicon-based sol-gel precursors.

In a preferred embodiment of the sixth aspect of the invention, the composition of the sixth aspect of the invention further comprises a solvent. Suitable solvents are those known in the art for the formation of polysiloxanes by the sol-gel method. Typically, suitable solvents are polar protic organic solvents such as alcohols. Such alcohols include, among others, methanol, ethanol, propanol, iso-propanol, butanol and *tert*-butanol. A preferred solvent is *iso*-propanol.

When the composition of the sixth aspect of the invention comprises a solvent, said solvent is preferably in an amount such that the ratio of the volume of the solvent to the volume of the silicon-based sol-gel precursors is from 1:2 to 2:1. More preferably, the solvent is in an amount such that the ratio of the volume of the solvent to the volume of the silicon-based sol-gel precursors is about 1:1.

An aspect falling outside the scope of the present invention relates to a composition for use in the prevention or treatment of inflammatory processes such as mucositis or periimplantitis, preferably of periimplantitis, wherein said composition is obtainable by a process comprising the steps of:

(i) providing a mixture comprising silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1\text{-}C_4)$alkylchain, preferably tetraethoxysilane, and a compound of formula $Si(R^5)(OR^6)(OR^7)(OR^8)$ wherein each of $R^5$, $R^6$, $R^7$ and $R^8$ is independently a $(C_1\text{-}C_4)$alkylchain, preferably methyltrimethoxysilane, in a molar ratio of from 80:20 to 50:50;
(ii) treating the mixture provided in step (i) with at least an effective amount of an aqueous acidic solution so as to form a sol material;
(iii) optionally, curing the sol material of step (ii) by heating said material at a temperature of about mouth temperature. The product obtainable according to said process also forms part of the invention.

In preferred embodiments of said aspect falling outside the scope of the invention, the inflammatory process susceptible of being treated or prevented by the compositions of the invention is one caused by one or more bacteria. Preferably, said one or more bacteria is one of the *Staphylococcus genus; particularly, Staphylococcus aureus, or is one of the Streptococcus genus; particularly, Streptococcus Gordonii,* or is selected from the group consisting of *Bacteroides, Campylobacter, Eubacterium, Fusobacterium, Treponema species, Aggregatibacter actinomycetemcomitans, Prevotella, intermedia, Porphyromonas gingivalis, Treponema denticola,* and *Tannerella forsythia.* In more particular embodiments, said one or more bacteria is *Staphylococcus aureus.* In other more particular embodiments, said one or more bacteria is *Streptococcus Gordonii.*

In a preferred embodiment of said aspect falling outside the scope of the invention, the composition does not comprise an active pharmaceutical ingredient, such as an antibacterial agent, particularly octenidine. In a preferred embodiment of the seventh aspect of the invention, the composition does not comprise a bioactive particle, a drug or/nor a peptide. In a preferred embodiment of the seventh aspect of the invention, the composition does not comprise an antibacterial agent such as octenidine, a bioactive particle, a drug or/nor a peptide. In a particular embodiment of the seventh aspect of the invention, the composition is obtainable by a process as described above which consists of said steps (i) to (iii).

In further preferred embodiments of said aspect falling outside the scope of the invention, the composition is one wherein the silicon-based sol-gel precursors of the mixture of step (i) and their relative amounts are as defined in any of the preferred embodiment of the first aspect of the invention defined above.

In further preferred embodiments of said aspect falling outside the scope of the invention, the composition is one wherein the mixture of step (i) comprises a solvent as defined in any of the preferred embodiment of the first aspect of the invention defined above. Preferably, said solvent is in an amount as defined in any of the preferred embodiment of the first aspect of the invention defined above.

In further preferred embodiments of said aspect falling outside the scope of the invention, step (ii) is as defined in any of the preferred embodiment of the first aspect of the invention defined above defining step (ii).

In further preferred embodiments of said aspect falling outside the scope of the invention, step (iii) is as defined in any of the preferred embodiment of the first aspect of the invention defined above defining step (iii).

In a further aspect falling outside the scope of the invention, the invention relates to a composition for use in the prevention or treatment of inflammatory processes such as mucositis or periimplantitis, preferably of periimplantitis, wherein said composition is obtainable by a process comprising the steps of:

(i) providing a mixture comprising silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1\text{-}C_4)$alkylchain, preferably tetraethoxysilane, and a compound of formula $Si(R^5)(OR^6)(OR^7)(OR^8)$ wherein each of $R^5$, $R^6$, $R^7$ and $R^8$ is independently a $(C_1\text{-}C_4)$alkylchain, preferably methyltrimethoxysilane, in a molar ratio of from 80:20 to 50:50, the mixture further comprising an antibacterial agent in an effective amount;
(ii) treating the mixture provided in step (i) with at least an effective amount of an aqueous acidic solution so as to form a sol material;
(iii) optionally, curing the sol material of step (ii) by heating said material at a temperature of about mouth temperature. The product obtainable according to said process also forms part of the invention. The antibacterial agent may be as described above.

In said further aspect falling outside the scope of the invention, the invention thus also relates to a method for the for the prevention or treatment of inflammatory processes such as mucositis or periimplantitis, preferably of periimplantitis; comprising administering to a patient in a need thereof an effective amount of a composition obtainable by a process comprising the steps of:

(i) providing a mixture comprising silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1\text{-}C_4)$alkylchain, preferably tetraethoxysilane, and a compound of formula $Si(R^5)(OR^6)(OR^7)(OR^8)$ wherein each of $R^5$, $R^6$, $R^7$ and $R^8$ is independently a $(C_1\text{-}C_4)$alkylchain, preferably methyltrimethoxysilane, in a molar ratio of from 80:20 to 50:50, the mixture further comprising an antibacterial agent in an effective amount;
(ii) treating the mixture provided in step (i) with at least an effective amount of an aqueous acidic solution so as to form a sol material;
(iii) optionally, curing the sol material of step (ii) by heating said material at a temperature of about mouth temperature. The product obtainable according to said process also forms part of the invention. The antibacterial agent may be as described above.

In said further aspect falling outside the scope of the invention, the invention thus also relates to the use of a

composition for the preparation of a medicament for the prevention or treatment of inflammatory processes such as mucositis or periimplantitis, preferably of periimplantitis, wherein said composition is obtainable by a process comprising the steps of:

(i) providing a mixture comprising silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1-C_4)$alkylchain, preferably tetraethoxysilane, and a compound of formula $Si(R^5)(OR^6)(OR^7)(OR^8)$ wherein each of $R^5$, $R^6$, $R^7$ and $R^8$ is independently a $(C_1-C_4)$alkylchain, preferably methyltrimethoxysilane, in a molar ratio of from 80:20 to 50:50, the mixture further comprising an antibacterial agent in an effective amount;
(ii) treating the mixture provided in step (i) with at least an effective amount of an aqueous acidic solution so as to form a sol material;
(iii) optionally, curing the sol material of step (ii) by heating said material at a temperature of about mouth temperature. The product obtainable according to said process also forms part of the invention. The antibacterial agent may be as described above.

In further preferred embodiments of said further aspect falling outside the scope of the invention, the composition is one wherein the silicon-based sol-gel precursors of the mixture of step (i) and their relative amounts are as defined in any of the preferred embodiment of the first aspect of the invention defined above.

In further preferred embodiments of said further aspect falling outside the scope of the invention, the composition is one wherein the mixture of step (i) comprises a solvent as defined in any of the preferred embodiment of the first aspect of the invention defined above. Preferably, said solvent is in an amount as defined in any of the preferred embodiment of the first aspect of the invention defined above.

In further preferred embodiments of said further aspect falling outside the scope of the invention, step (ii) is as defined in any of the preferred embodiment of the first aspect of the invention defined above defining step (ii).

In further preferred embodiments of said further aspect falling outside the scope of the invention, step (iii) is as defined in any of the preferred embodiment of the first aspect of the invention defined above defining step (iii).

Throughout the description and claims the word "comprises" and variations of the word, are not intended to exclude other technical features, additives, components or steps. Furthermore, the word "comprise" encompasses the cases of "consist of" and "consists essentially of". Additional objects, advantages and features of the invention will become apparent to those skilled in the art upon examination of the description or may be learned by practice of the invention. The following examples are provided by way of illustration, and they are not intended to be limiting of the present invention.

## EXAMPLES

Example 1: Preparation and characterization of hybrid organic-inorganic materials as anti-infective coating compositions

General procedure for the preparation of a coating composition

Methyltrimethoxysilane (MTMOS) and ethyl tetraorthosilicate (TEOS) were mixed at different molar ratios as shown in Table 1 and dissolved in 2-propanol at 1:1 vol ratio. Octenidine was added to this solution at several different concentrations (1.0, 2.0, 5.0 and 7.5 wt%). A stoichiometric amount of an aqueous solution of nitric acid (0.1 N $HNO_3$) was added drop-by-drop to catalyse the sol-gel reaction. The solution was stirred for 30 minutes at 70 °C to allow the sol-gel polymerisation to take place by siloxane bonding. The resulting compound was further cured for another 10 minutes at 37 °C. Table 1 below summarizes the prepared materials:

Table 1

| Material | Molar ratio of TEOS:MTMOS | Content of octenidine (wt%) |
|---|---|---|
| M1 | 3:2 | 0.0 |
| M2 | 3:2 | 1.0 |
| M3 | 3:2 | 2.0 |
| M4 | 3:2 | 5.0 |
| M5 | 3:2 | 7.5 |
| C1 | 0:1 | 2.5 |
| C2 | 1:9 | 2.5 |

(continued)

| Material | Molar ratio of TEOS:MTMOS | Content of octenidine (wt%) |
|---|---|---|
| C3 | 2:8 | 2.5 |
| C4 | 3:7 | 2.5 |
| M6 | 1:1 | 0.0 |

As will be obvious to the skilled person, samples C1-C4 have molar ratios of TEOS:MTMOS outside the scope of the present invention. These materials are thus provided as comparative examples.

All samples M1-M5 were characterized by FT-IR spectroscopy. All samples exhibit absorption peaks at 1075 $cm^{-1}$, 1105 $cm^{-1}$ and 760 $cm^{-1}$, which correspond to vibration modes of the polysiloxane compound, which allows verifying that the gel material formed successfully. Also, the presence of peaks at 1655 $cm^{-1}$ and 2920 $cm^{-1}$ account for the presence of octenidine in the formed materials M2-M5. The intensity of said peaks increases with the concentration of octenidine in the material.

General Procedure for preparation of a coated surface:

A volume of a mixture of Methyltrimethoxysilane (MTMOS) and ethyl tetraorthosilicate (TEOS) at different molar ratios as shown in Table 1 was added to the same volume of a solution of octenidine in 2-propanol. The amount of octenidine was as indicated in Table 1 (1.0, 2.0, 5.0 and 7.5 wt%, being said weight% expressed over the weight of siloxane precursors). A stoichiometric amount of an aqueous solution of nitric acid (0.1 N $HNO_3$) was added drop-by-drop to catalyse the sol-gel reaction. The solution was stirred for 30 minutes at 70 °C to allow the hydrolysis and condensation of precursors to take place by siloxane bonding. Said solution was transferred to a SAE-Ti disc by dip-coating. The discs were immersed in the sol-gel solutions at a speed of 60 cm $min^{-1}$, left immersed for 1 min, and removed at a 100 cm $min^{-1}$. Additionally, the materials were also applied onto glass slides by casting to evaluate the hydrolytic degradation and the antibacterial agent release. Samples were then cured at 37 °C for a period of 10 minutes.

As shown on Figure 11 showing the results of a cross cut assay carried out on metal surfaces coated with M1, M2 or M5, the coating compositions are well adhered to the metallic surface, as no material appears to be detached from the surface of the metal.

Following a similar procedure, compositions C1, C2 and M6 were coated on steel 316L square pieces. The coated pieces were placed in an atmosphere of 100% humidity at 37 °C for 10 minutes. After this time, the top lateral side of the coated surface of pieces was wiped with a paper tissue in order to evaluate qualitatively the degree of curing and adhesion of the material to the surface of the substrate. As shown in Figure 12, sample M6 is the only sample whereby the coating is cured and adhered on the surface, as no material appears to be wiped out from the surface of the metal.

General procedure for hydrolytic degradation test

To obtain the samples for the hydrolytic degradation study, glass microscope slides were coated using the drop-casting method with a sol prepared according to the procedure described above. The resulting coated glass slide was heated at 37 °C for a period of 10 minutes to cure the coating. Prior to the preparation of the coated slides, the wettability of the slides was increased by ultrasonic cleaning. The slides were sonicated (Sonoplus HD 3200) in a solution of $HNO_3$ (25%, w/w) for 15 min, followed by cleaning the sonicated slides in distilled water three times. Finally, the slides were dried in an oven at 100 °C and stored in a desiccator.

The polysiloxane network degrades by hydrolysis in aqueous media as follows: $SiO_2$ (s) + 2 $H_2O \rightarrow Si(OH)_4$ (aq) Hydrolytic degradation was evaluated using gravimetric measurements, by comparing the weight of the coating compositions before and after soaking for different periods of time in distilled water at 37 °C. Samples of about 25 mg of each coating composition were used.

Figure 1 shows the results of said assay. The results show that the rate of hydrolytic degradation of materials comprising octenidine is faster than the rate of degradation of materials not comprising octenidine (M1).

General procedure for measurement of rate of octenidine release

The amounts of antimicrobial agents released during the degradation of the network were evaluated using UV-visible spectrometry. Coating samples of 1 g were immersed in 50 mL of distilled water and left in an incubator at 37 °C for up to 1 month. At several time points, a 5 mL-sample was removed, and the biocide concentration was determined. The absorbance was measured at $\lambda$=282 nm for octenidine. A linear calibration curve was obtained for octenidine. Measurements were performed in triplicate.

Figure 2 shows the kinetics of release of octenidine with materials M2-M5. Figure 8 shows the kinetics of release of octenidine with comparative materials C1-C4.

The results of Figure 2 show that materials M2-M5 behave similarly in releasing octenidine. The release of octenidine is sustained at least to 14 days. This is advantageous as it allows:

(i) Providing the anti-infective agent during the first days following the surgery, when it is particularly needed to prevent or treat the development of the infection eventually caused by the surgical act, and
(ii) Gradually releasing the anti-infective agent after a few days to prevent or treat the development of new infections.

It is also deduced from the data of Figure 2 that the amount of octenidine released gets faster when the gel material has increasing concentrations of octenidine.

In addition, when the material comprises at least 2% in weight of octenidine, the concentration of octenidine released in the medium is above 2 mg/L in the conditions of the assay from two hours of lapsed time. It is particularly advantageous, for such concentration is known in the art as being above both the minimal inhibitory concentration (MIC) and minimal bactericide concentration (MBC) for *Staphyloccocus Aureus* and is thus suitable for eradication of bacteria.

Furthermore, the results of Figure 8 show that increasing the proportion of TEOS in a sol-gel material according to the prior art provides an increasingly immediate release of octenidine, due to the more hydrophilic character of the formed material which makes it more prompt to hydrolytic degradation. For instance, composition C2 (10% TEOS) shows that practically all of the octenidine released at 28 days is released at 14 days; composition C3 (20% TEOS) shows that practically all of the octenidine released at 28 days is released at 7 days; composition C4 (30% TEOS) shows that practically all of the octenidine released at 28 days is released at 3 days..

However, composition M3 according to the present invention (60% TEOS) shows a sustained release of octenidine up to day 14. This, in view of the results of comparative compositions C2 to C4 of Figure 8, is totally unexpected, for a reduction in the release period of octenidine would have been expected upon further increasing the amount of TEOS, rather than a prolongation. Thus, the compositions according to the present invention are unexpectedly capable of releasing large amounts of octenidine without sacrificing release rate.

The sustained release of octenidine advantageously allows providing anti-infective agent for a longer period of time and has a positive effect on the prevention or treatment of periimplantitis as it allows delivering an anti-bacterial agent at least during 14 days after the surgery or the application of the coating in order to avoid the development of new infections and prevent recurrence of infection.

General procedure for cell viability assay

*Cell culture:* Human fibroblasts (HFB) and human osteoblasts (MG-63) were employed to evaluate in vitro the materials. Cells were cultured at 37 °C in a humidified (95%) $CO_2$ incubator in Dulbecco's Modified Eagle Medium (DMEM) supplemented with 1% penicillin/streptomycin and 10% FBS. The coated Ti discs were sterilized prior to cell culture by exposure to UV radiation for 30 min. Ti samples without coatings were employed as controls.

Cytotoxicity was evaluated using the 3-(4,5-dimethylthiazol-2-yl)-2,5-diphenyltetrazolium bromide (MTT) assay. Titanium disc samples coated with the different compounds, Ti were submerged in 5 mL of culture medium and placed on a shaker at 37 °C. The medium was removed after different periods of time (2, 7, 14 and 21 days) and replaced with 5 mL of fresh medium. All the extracts were obtained under sterile conditions and stored frozen. Meanwhile, the cells were seeded at a density of $9\times10^4$ cells/mL in complete medium in a sterile 96-well culture and incubated at 37 °C in humidified air with 5% $CO_2$ for 24 h. To test the toxicity of the extracts, the medium was replaced with the corresponding extracts and cultures incubated for another 24 h. Cell viability was analysed after adding a solution of MTT (0.5 mg/mL) in PBS and incubating at 37 °C for 3 h. Excess medium and MTT were removed and washed with PBS. Dimethyl sulfoxide was added to solubilise the formazan crystals formed in viable cells. The mixture was stirred for 10 min, and the absorbance was measured using a Biotek Synergy HT detector at a wavelength of 570 nm. The percentage of cell viability was calculated as follows:

$$\text{Cell viability (\%)} = 100 \times (ODS - ODB)/(ODC - ODB),$$

where ODS, ODB and ODC are the optical densities of formazan product for the sample (S), blank (B) (culture medium without cells) and control (C), respectively.

Figure 3 shows the cell viability of human fibroblast cells (FBH, expressed as a percentage) in function of time ((a): 1 day, (b): 2 days. (c): 7 days)) put in contact with samples M1-M5 as prepared in Example 1 and measured according to MTT cell viability assay. The results of Figure 3 show that the materials M1-M5 do not exhibit cytotoxicity as the cell viability is above 70% even after 7 days.

Figure 4 shows the cell viability of human osteoblast cells (MG-63, expressed as a percentage) in function of time ((a): 1 day, (b): 2 days. (c): 7 days)) put in contact with samples M1-M5 as prepared in Example 1 and measured according

to according to MTT cell viability assay.

General procedure for adhesion assays

Titanium disc (with and without coating) were placed in a 24-well plate, $10^5$ FBH or MG-63 cells/mL were seeded on the discs and incubated (37 °C with 5% CO2) for 24 h. The different discs were transferred to a new 24-well plate to exclude the cells adhering to the well. The medium was removed after different periods (1, 2 and 7 days) and cell adhesion and proliferation on the discs were quantified using alamarBlue assay. The cells were incubated with 10% solution of alamarBlue in fresh medium without phenol red. After 3 h, the solution was transferred to a 96-well plate and replaced with fresh medium. The fluorescence of the transferred media was measured at 530 nm (excitation) and 600 nm (emission) on a UV Biotek Synergy HT detector. The average baseline fluorescence of control media without cells (negative control) was subtracted to obtain the sample fluorescence. All experiments were repeated five times, and the one-way ANOVA test was performed (relative to Ti) to determine significant differences between the results.

Figures 5 and 6 show the cell proliferation of human fibroblast cells (FBH) and human osteoblast cells (MG-63) respectively put in contact with samples M1-M5 in function of time.

General procedure for measurement of bactericide effect

Antibacterial activity of the materials was determined following the method described in the standard ISO 22196:2011, using Staphylococcus aureus CECT 86. The bacterial inoculum was obtained as follows: precultures were grown overnight at 37 °C in Luria Broth (LB; 0.5% yeast extract, 1% tryptone and 1% NaCl, all supplied by Difco Laboratories, Detroit, MI, USA) until the stationary phase. Bacterial stock solutions were prepared from the pre-cultures; an aliquot was taken with a sterile inoculating loop and diluted with PBS to obtain the optical density of 0.1 at 550 nm (OD550=0.1) (A600 BOECO S-22 spectrophotometer). This value corresponds to a bacterial concentration of $1.6\times10^7$ CFU/ mL (interpolating the A550 values in a standard curve of CFUs versus optical density). The final test inoculum was obtained by re-suspending the stock solution with 1/500 LB to the concentration of $6\times10^5$ CFU/ mL.

Titanium discs (ø=16 mm) coated with M1-M5 were seeded with 100 µL of test inoculum and incubated for 24 h at 37 °C, in a humidified atmosphere. The samples were washed several times with 900 mL of PBS; this suspension was used for the determination of the final CFUs using standard agar diffusion method. The reported results are relative to control values (titanium disc without coating). The test was performed in triplicate.

The results were expressed as relative cell viability (RCV):

$$RCV(\%) = N/Ncontrol \times 100$$

where N is the number of CFUs on 1 $cm^2$-sample.

All data are expressed as means ± standard deviations. Statistical analyses were performed using one-way ANOVA. Values of $*p < 0.05$ and $**p < 0.001$ indicated significant differences.

Figure 7 shows the results of the tests carried out. A clear bactericide effect is visible when the material comprises at least 1% weight octenidine.

Protein layer elution and proteomic analysis

The protein layers on the distinct sol-gel formulations were examined after their incubation in a humidified atmosphere (37 °C, 5 % $CO_2$) for 3 h with 1 mL of human blood serum from male AB plasma (Sigma-Aldrich). The serum was removed, and non-adsorbed proteins were eliminated by five consecutive washes with dd$H_2O$ and another with 100 mM NaCl, 50 mM Tris-HCl, pH 7.0. The adsorbed proteins were eluted by washing the surfaces with 0.5 M triethylammonium bicarbonate buffer (TEAB), with 4 % sodium dodecyl sulfate (SDS) and 100 mM-dithiothreitol (DTT). The experiment was performed in quadruplicate for each material, and each of these replicas was the result of pooling four different processed samples.

The eluted proteins were characterized using electrospray tandem mass spectrometry, employing a nanoACQUITY UPLC (Waters, Milford, MA, USA) coupled to an Orbitrap XL (Thermo Electron, Bremen, Germany). The protocol described by Romero-Gavilán et al. Biofouling 2017, vol. 33(8), p. 676-689 was followed. Each condition was analyzed in quadruplicate. Proteomic results were examined using PEAKS (Bioinformatics Solutions Inc., Waterloo, Canada). For statistical analysis, Student's t-test was performed using PEAKS, and protein adsorption differences were considered statistically significant at $p \leq 0.05$ and a ratio higher than 1.5 in either direction.

Table 2 shows a proteomic comparative analysis between proteins adsorbed onto the sol-gel composition M3 with 2% octenidine with respect to the control M1 (0% octenidine).

| | | M3 vs M1 | |
|---|---|---|---|
| Accession | Unique peptides | p value | Ratio |
| CO4A_HUMAN | 1 | 0,28 | 2,76 |
| PROS_HUMAN | 3 | 0,23 | 2,15 |
| ITIH1_HUMAN | 3 | 0,24 | 1,95 |
| APOF_HUMAN | 3 | 0,26 | 1,94 |
| CLUS_HUMAN | 10 | 0,38 | 1,92 |
| C4BPA_HUMAN | 6 | 0,14 | 1,9 |
| HEP2_HUMAN | 4 | 0,45 | 1,77 |
| CD5L_HUMAN | 5 | 0,36 | 1,72 |
| IGHM_HUMAN | 6 | 0,31 | 1,69 |
| ITIH3_HUMAN | 4 | 0,32 | 1,67 |
| AACT_HUMAN | 8 | 0,54 | 1,64 |
| AMBP_HUMAN | 6 | 0,35 | 1,59 |
| CO5_HUMAN | 11 | 0,21 | 1,58 |
| THRB_HUMAN | 7 | 0,77 | 1,5 |
| ITIH2_HUMAN | 5 | 0,43 | 1,49 |
| IC1_HUMAN | 4 | 0,25 | 1,48 |
| CERU_HUMAN | 8 | 0,67 | 1,46 |
| IGJ_HUMAN | 2 | 0,14 | 1,45 |
| APOD_HUMAN | 4 | 0,54 | 1,44 |
| CFAH_HUMAN | 20 | 0,43 | 1,34 |
| APOA4_HUMAN | 19 | 0,07 | 1,33 |
| VTNC_HUMAN | 8 | 0,18 | 1,33 |
| C1S_HUMAN | 8 | 0,33 | 1,29 |
| GPX3_HUMAN | 2 | 0,97 | 1,27 |
| ITIH4_HUMAN | 5 | 0,58 | 1,21 |
| C1R_HUMAN | 6 | 0,61 | 1,2 |
| APOB_HUMAN | 58 | 0,99 | 1,19 |
| APOC2_HUMAN | 2 | 0,54 | 1,19 |
| SAMP_HUMAN | 5 | 0,44 | 1,18 |
| ANT3_HUMAN | 5 | 0,60 | 1,17 |
| APOM_HUMAN | 2 | 0,63 | 1,11 |
| APOA_HUMAN | 4 | 0,65 | 1,08 |
| APOL1_HUMAN | 4 | 0,67 | 1,04 |
| SEPP1_HUMAN | 3 | 0,95 | 1,01 |
| PROC_HUMAN | 2 | 0,86 | 0,95 |
| SAA4_HUMAN | 3 | 0,38 | 0,83 |
| CXCL7_HUMAN | 2 | 0,16 | 0,51 |

The results of Table 2 show that a composition according to the present invention is biocompatible and suitable for in vivo applications, as there is no variation of statistical relevance between the p values obtained for samples M3 and M1.

Indeed, it is considered that a variability of between 0 % and 2% is of no statistical relevance.

Clinical trials and results

A comparative study of uncoated titanium implants and titanium implants coated with material M3 has been carried out by introduction of said implants in the tibia of New Zealand white rabbits *Oryctolagus* cuniculus. The implants used in this study were obtained from GMI Dental Implantology, and are suitable for titanium-made dental implants with internal connection having a diameter of 3.75 mm and a length of 8 mm. The coated implants were prepared in a white room by dip coating of the implant in the sol prepared as described above and cured following the procedure described above. Prepared implants were packed and sterilized via UV light irradiation prior to the clinical study.

Implants were introduced by surgery as follows: female rabbit were weighted and administered the corresponding dose of a mixture of ketamine and xylazine as pre-anaesthetics, followed by a dose of torbugesic and prophylactic antibiotic treatment. Propofol was administered as sedative agent and was administered in a continuous manner during surgery. Tibia bones were perforated using a progressive milling technique by means of drills of growing diameter until reaching the diameter of the implant. Control and test implants were inserted in the right and left legs of the animal respectively. Animals were sacrificed 4 weeks past the surgery.

Once the animals were sacrificed, the tibiae were sectioned using a saw for osteotomy and submerged in 40% ethanol at 4°C for fixation and conservation. The samples were dehydrated using increasing solutions of ethanol under vacuum until reaching absolute ethanol and once dehydrated, the ethanol in the tissues was replaced by xylene. Samples were then inserted in polymethylmethacrylate matrices in order to preserve the mineral phases and enable the observation of cementing lines using taints. The insertion in the matrix consists in submerging the samples in solutions of methyl methacrylate (monomer, Sigma-Aldrich), dibutyl phthalate (plasticizer, Sigma-Aldrich) and growing concentrations of benzoyl peroxide as initiator. Samples were finally introduced in glass flasks and, once the monomer was added, the mixture was polimerized by heating at 40 °C. Resulting samples were then cut (EXAKT®) and treated with taints (Gomori trichrome). Tainted samples allow distinguishing growing bone and osteoid (red tones) from more mature bone tissue (blue tones). Fibrous tissue and cellular cytoplasm also get tainted in red and can be recognized by their morphology.

The response to the presence of an external body, such as an implant, has been assessed via subjective and semi-quantitative assessment of the inflammatory response associated to the presence of the implant. This response is a preparative reaction of the organism faced with an exogenous body aiming at isolating said exogenous body from the organism and, if possible, reject it. The response of the organism to the presence of an external body is a non-allergic inflammatory reaction whereby histiocytes and giant cells intervene to isolate the external body, followed by the intervention of phagocytes.

The response of the bone to the implant is a natural response that takes place to achieve the repair of the injury, its prolongation in time will secondarily trigger a fibrosis reaction. It is determined by evaluating 12 factors, from 0 to 3, depending on whether they are not detected or their frequency is mild, moderate or severe. A semi-quantitative evaluation system based on ISO 10993-6 (2009) was employed, and 5 categories of parameter were evaluated:

1. Bone marrow condition (defined as the level of trauma to bone); aplasia (1), loss of architecture (2), appearance in areas of the marrow not in contact with the implant (3), fat ratio (4).
2. Necrosis, defined as the level of trauma to cortical bone (5) and trabecular bone (6) caused by effects of implantation.
3. Presence of giant foreign body reaction cells (7), attached to the different surfaces (titanium in the case of control and coating in the case of sol-gel materials).
4. Presence of fibrosis/fibrous capsule in the marrow (8), between the cortical bone and the implant (9), between the trabecular bone and the implant (10) and degree of densification (11).
5. Neovascularization (12). Based on the score obtained, it was determined whether the implant was non-irritating (0-6), slightly irritating (6-18), moderately irritating (18-30), and severely irritating (30-36).

*Results of biocompatibility evaluation*

The biocompatibility of a material can be defined as the biological acceptability of the same by the body. This biological acceptability can be examined from several levels, one of them being the interaction between the material and the surrounding tissues. For this reason, in the development process of a biomaterial it is essential to study the effects induced on the adjacent tissues after its implantation in vivo. For this, it was verified that the new coating compositions developed did not induce an adverse reaction on the tissues, as shown in Figures 9 and 10.

*Formation of fibrous capsule*

As shown in Figure 13, the coating of an implant with a coating according to the invention made from silicon-based precursors with high content of tetra(alkoxy)silane compound(s) allows preventing the formation of a fibrous capsule. It is in particular observed that, when a coating with a low content of tetra(alkoxy)silane (e.g. 10% of TEOS) is used on an implant, a fibrous capsule is formed (as represented by an * symbol on Fig. 13), leading to rejection of the implant. Such capsule is surprisingly not observed when the implant is coated with a material according to the invention.

Clinical trials in beagle dogs

The research on the pathogenesis and treatment of periimplantitis was carried out in experimental studies carried out in model animals where periimplantitis was developed in the hard and soft tissues that support the implants. This was achieved by the placement of ligatures in the periimplant sulcus and the subsequent abandonment of hygienic plaque control measures.

*Experimental procedure of clinical trials*: 8 adult female beagle dogs were submitted to the following steps:

(1) extraction of mandibular premolars;
(2) insertion of internal connection implants with sandblasted and etched surface Frontier ® in lieu of mandibular premolars (GMI, Barcelona, Spain) - 3 months after step 1 (T0 samples correspond to mucosa samples taken right before the insertion of the implants);
(3) development of periimplantitis by ligature of implants and abandonment of hygienic plaque control measures- 3 months after step 2; (T3 samples correspond to mucosa samples taken right before the ligature of implants);
(4) withdrawal of ligatures - 3 months after step 3 (T6 samples correspond to mucosa samples taken right after the withdrawal of ligatures);
(5) examination of periiimplant tissues and randomized assay for treatment with the composition M3 of the implants or with a placebo - 1 month after step 4; (T7 samples correspond to mucosa samples taken right before said treatment);
(6) examination of periiimplant tissues and implants - 3 months after step 5; (T10 samples correspond to mucosa samples taken 3 months after the treatment of step (5))
(7) animal sacrifice to isolate bon samples - 3 months after step 6.

*Histological study of the periimplant mucosa*

Samples at times T0, T3, T6, T7 and T10 obtained from the dogs were included in paraffin and samples of 4 μm thickness were cut and tainted with Haematoxylin-Eosin according to standard procedures for microscope observation. Different areas of the periimplant mucosa were found of particular interest for the histological study, as shown on Figure 14: epithelium (E), infiltrated connective tissue in connective papillae (TCI1) and infiltrated connective tissue in areas remote from the epithelium (TCI2). Inflammatory infiltration, related to the presence of plasmatic cells, lymphocytes, neutrophils and macrophages in said areas was rated qualitatively according to the following scale: 0 - no infiltration, 1- little infiltration, 2- moderate infiltration, 3- abundant infiltration.

Figure 15 shows a microscope picture of a mucosa sample at T0.

Figure 16 shows a microscope picture of a mucosa sample at T3.

Figure 17 shows a microscope picture of a mucosa sample at T6.

Figure 18 shows a microscope picture of a mucosa sample at T7.

Figure 19 shows a microscope picture of a mucosa sample at T10.

Table 3 below summarizes the ratings attributed to the samples taken for each tested dog at different times of the assay in different areas of the mucosa (TCI1 and TCI2):

Table 3

| Dog # | T0 | | T3 | | T6 | | T7 | | T10 | |
|---|---|---|---|---|---|---|---|---|---|---|
| 1824 | TCI10 | TCI20 | TCI11 | TCI22 | TCI13 | TCI22 | TCI11 | TCI21 | TCI11 | TCI21 |
| 3255* | TCI10 | TCI21 | TCI10 | TCI20 | TCI12 | TCI23 | TCI12 | TCI23 | TCI10 | TCI21 |
| 8718* | TCI10 | TCI20 | TCI11 | TCI22 | TCI12 | TCI22 | TCI12 | TCI23 | TCI10 | TCI21 |
| 7070 | TCI101 | TCI20 | TCI11 | TCI23 | TCI12 | TCI23 | TCI11 | TCI23 | TCI11 | TCI23 |
| 1668* | TCI10 | TCI20 | TCI13 | TCI23 | TCI11 | TCI23 | TCI13 | TCI23 | TCI10 | TCI21 |
| 8294* | TCI10 | TCI20 | TCI11 | TCI23 | TCI13 | TCI23 | TCI12 | TCI23 | TCI11 | TCI21 |

(continued)

| Dog # | T0 | | T3 | | T6 | | T7 | | T10 | |
|---|---|---|---|---|---|---|---|---|---|---|
| 2402 | TCI10 | TCI20 | TCI11 | TCI23 | TCI13 | TCI23 | TCI12 | TCI23 | TCI11 | TCI21 |
| 5590 | TCI10 | TCI20 | TCI10 | TCI21 | TCI11 | TCI23 | TCI13 | TCI23 | TCI10 | TCI21 |
| * dog treated with mixture M3 | | | | | | | | | | |

The results of Table 3 show that the inflammatory process can be reverted, in particular in areas close to the epithelium upon treatment of the implant with the mixture M3.

Immunohistochemical studies of the above samples was also performed in order to detect the presence of biomarkers related to the inflammatory response, said biomarkers being P substance and NKx1 receptor. To do so, samples were removed from paraffin and hydrated to be subsequently heated at pH 6 (citrate buffer). Endogenous peroxidase was then inhibited and the samples were treated with normal donkey serum. Samples were then incubated overnight with substance P rat monoclonal antibody (bio-Rad, rat monoclonal, clone NC1/34; dilution 1:50 vol/vol) and anti Neurokinin-1 receptor (bioss rabbit polyclonal antibody, dilution 1:50 vol/vol). LSAB system (vectastain, Véctor) and diaminobenzidine (Sigma-Aldrich) were used as chromogens.

Receptor NKx1 can be identified in all the samples. is identified in all samples; however, the extent and intensity of Staining is greater as the inflammatory response increases, while being weaker in pre-implant samples. NKx1 is expressed in the epithelium, endothelial cells, nerve pathways, leukocytes, macrophages and plasma cells.

The amount of substance P increases together with chronic inflammatory response. As shown in Figure 20, a relationship is observed between substance P and inflammatory response, the expression of substance P being reverted upon treatment of the dog with mixture M3.

*Histological results of the bone*

Bone samples obtained from step (7) were placed in a 10% vol/vol formaline solution. The jaws of the dogs were sent intact in vials with a numerical code that identified the animal. Once the implants separated in individual containers, samples were initially identified with a number that were the last 4 digits of the dog code, R or L depending on which side left or right and a number that identified it from mesial to distal. The samples were prepared following the method described by Donath & Breuner, J. Oral Pathol. Med. 11, 318-326 (1982). Briefly, the samples were dehydrated in alcohol at increasing concentrations and infiltrated in mixtures of ethanol and methacrylate glycol (Technovit 7200 VLC, Heraus Kulzer, Werheim, Germany). They were subsequently polymerized by immersion in pure resin (Technovit 7200) using a specific light source and heated at 37°C for 24 hours to ensure complete polymerization.

A central cut of each implant was then performed by (i) taking a radiography of each sample and (ii) cutting with a hacksaw of diamond band (Exakt Apparatebau, Norderstedt, Germany). Samples were then polished till having a defectless flat surface. Then the block was glued to a definitive slide using Technovit 7210, a section of about 200 microns was made and reduced by micropolishing (Exakt Apparatebau, Norderstedt, Germany) using 1200 to 4000 grit silicon carbide polishing cloths (Struers,Copenhagen, Denmark). When they reached about 40 microns thick, they were all dyed together using the Levai-Laczkó method. This stain technique combines two blues (Methylene Blue and Azure II) with a basic fuchsin (violet). Staining is commonly used for bone embedded in resin. Bone stains pink, cartilage violet, and collagen fibers blue-violet. The slides were scanned using a motorized optical microscope and a digital camera connected to a computer (BX51, DP71, Olympus Corporation, Japan). The images were captured at x40 increase.

The analysis was performed by a researcher blind to the treatment groups used, which were only revealed once the analysis was completed. The measurements were obtained using a Cell-sens 1.5 image analysis program (Olympus Corporation, Japan).

The results proved that implants from the control group presented bone resorptions in the areas in contact with the implant at a coronal level, which is usually accompanied by a greater number of cells of the lymphocyte series indicating a chronic inflammatory process. This leads to the observation of a pocket around the implant. This phenomenon was also observed in a lesser extent in the group having received the treatment. This fact has been studied when making saucerization measurements.

The results of Figure 21 show that the treatment with the coating allows regeneration of the soft tissue with no or little formation of an inflammation-related pocket around the implant, thus evidencing the benefits of the coating in preventing the development of inflammatory processes, such as periimplantitis.

Mechanism of action

The mechanism of action of the composition of Example 1 was further investigated by studying in parallel assays

the proliferation of bacterial population of *Streptococcus Gordonii* on grade- IV titanium plates which are either uncoated or coated with M1 (free of octenidine) or M3 (2 % in weight of octenidine) or with a solution of octenidine having an equivalent amount of octenidine as the composition M3. The following experimental procedure was used.

All materials used were sterile or have been previously sterilized by autoclaving. The assays were performed inside a level 2 biosafety cabinet. Three different grade IV-Ti disks were used to perform the assay. This procedure was carried out for each of the assays: First, a suspension of *Streptococcus Gordonii* (CECT 804) of known concentration was prepared. For this purpose, a test tube is prepared with 10 mL of d.d. $H_2O$ to which bacteria were added until the desired concentration (Concentration=1 x$10^6$ CFU/mL). These data were verified by direct measurement by spectrophotometry at 600nm. 500μL of bacterial suspension of known concentration (1 x$10^6$ CFU/mL) was then added in wells of a sterile 24-well plate. Subsequently, the grade-IV Ti disc was immersed in the bacterial suspension for 1 minute (1 disk per well) in order to inoculate the disc. Once inoculated, the grade-IV Ti disc was immersed for 1 min in 500μL of mixture M1, mixture M3 or octenidine solution (1 disk per well - a control experiment with no mixture at all was also carried out for control purposes). This procedure was done 3 times per disc. Once coated, grade-IV Ti disc was cured in a sterile 24-well plate. When the product grade-IV Ti disc was cured, the grade-IV Ti disc was placed on a sterile agar plate. This procedure was done with 3 different plates (1 disk per well). Finally, and following the culture protocols of *Streptococcus Gordonii,* the agar plates were incubated at 37°C for 24h for a correct proliferation of the bacteria.

Once this time was elapsed, bacteria population was counted. For the bacterial count, inoculated grade-IV Ti disc were removed. After that, 3 mini-discs (extracted in the same place where the inoculated discs were placed) of known diameter per plate were extracted using a punch. Once the minidiscs were obtained, they were taken to a falcon tube with 1 mL of d.d.$H_2O$ and passed through the vortex, in order to suspend the bacteria adhered to the agar. Once the suspension was in the falcon, CFU adhered to the agar were obtained following this protocol: a) From the bacterial suspensions obtained, 9 serial dilutions (1:10) were performed for each replicate and condition; b) Each of these dilutions (100μL per agar plate) were added to sterile agar plates; c) Once added, they were taken to the incubator for 24h at 37°C; d) After this time, the CFU count of those plates in which it was possible to count between 30-300 colonies was carried out.

The results of Fig. 22 show that bacteria population did not proliferate at all after 24 hours incubation on the disks coated with M1 or M3, while a significant reduction of bacteria proliferation was observed for the disk coated with the octenidine solution.

Bacterial count of bacteria adhered to the titanium surface was carried out in these samples. The average results (three samples were measured for each disk) are shown in Table 9:

Table 9

| Coating | CFU/mL |
|---|---|
| none | 1,726 x $10^7$ |
| octenidine | 483,33 |
| M1 | 0 |
| M3 | 0 |

This Experiment shows that M1 and M3 are suitable for preventing the proliferation of bacterial population on the surface of a substrate coated with M1 or M3. Surprisingly, the coating of an implant substrate with a composition deprived of antibacterial agent such as composition M1 allows the prevention of the adhesion of bacteria to the surface of said substrate. Octenidine alone does not allow reaching such an effect, as some bacteria proliferation is still observed. Thus, without being bound to any theory, it is believed that the sol-gel precursors of the mixture form upon curing a physical barrier sealing the substrate site against the possible entry of bacteria. It is also believed that the film is also suitable for embedding the bacteria adhered to the substrate surface, thus resulting in the elimination of bacterial population adhered to the surface of the substrate. In some embodiments, the physical barrier is robust enough to allow for regeneration of soft tissue around the implant, thus preventing efficiently the development of an infection-related inflammatory response around the implant.

In addition, octenidine is believed to have an ancillary action and provide anti-bacterial effect once released from the cured sol-gel material. as a physical barrier between bacteria from the physiological medium and the surface of the implant.

## Claims

1. A process for the preparation of a composition comprising the steps of:

(i) providing a mixture comprising silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1\text{-}C_4)$alkyl chain, and a compound of formula $Si(R^5)(OR^6)(OR^7)(OR^8)$ wherein each of $R^5$, $R^6$, $R^7$ and $R^8$ is independently a $(C_1\text{-}C_4)$alkyl chain, the molar ratio compound of formula $Si(OR^1)(OR^2)(OR^3)(OR^4)$ : compound of formula $Si(R^5)(OR^6)(OR^7)(OR^8)$ being from 80:20 to 50:50, the mixture further comprising octenidine in an amount of from 1 to 7.5 grams per each 100 grams of the silicon-based sol-gel precursors;
(ii) treating the mixture provided in step (i) with an aqueous acidic solution so as to form a sol material;
(iii) optionally, curing the sol material of step (ii) by heating said material at a temperature of about mouth temperature.

**2.** The process according to claim 1 wherein the compound of formula $Si(OR^1)(OR^2)(OR^3)(OR^4)$ is tetraethyl orthosilicate and the compound of formula $Si(R^5)(OR^6)(OR^7)(OR^8)$ is methyltrimethoxysilane.

**3.** The process according to any one of claims 1 to 2 wherein the molar ratio of the compound of formula $Si(OR^1)(OR^2)(OR^3)(OR^4)$ to the compound of formula $Si(R^5)(OR^6)(OR^7)(OR^8)$ is about 60:40.

**4.** The process according to any one of claims 1 to 3 wherein the silicon-based sol-gel precursors of the mixture of step (i) comprise the compound of formula $Si(OR^1)(OR^2)(OR^3)(OR^4)$ and the compound of formula $Si(R^5)(OR^6)(OR^7)(OR^8)$ in an amount of at least 80% by weight with respect to the total weight of the silicon-based sol-gel precursors.

**5.** The process according to any one of claims 1 to 4 wherein the silicon-based sol-gel precursors of the mixture of step (i) do not comprise glycidoxypropyltrimethoxysilane.

**6.** The process according to any one of claims 1 to 5 wherein the mixture of step (i) comprises octenidine in an amount of from 1 to 5 grams per each 100 grams of the silicon-based sol-gel precursors.

**7.** The process according to any one of claims 1 to 6 wherein the aqueous acidic solution of step (ii) is a solution of nitric acid in a concentration of from 0.01 N to 0.5 N.

**8.** The process according to any one of claims 1 to 7 wherein step (ii) is carried out at a temperature of between 50 °C and 100 °C.

**9.** The process according to any one of claims 1 to 8 wherein step (iii) is carried out.

**10.** Composition obtainable by the process as defined in any one of claims 1 to 9.

**11.** Composition according to claim 10 for use in medicine.

**12.** Composition according to claim 10 for use in the prevention or treatment of inflammatory processes.

**13.** Composition for use according to claim 12 wherein said inflammatory process is periimplantitis.

**14.** A kit of parts for preparing a substrate coated with an anti-infective coating composition comprising:

- in a first part, a mixture as provided in step (i) of the process defined in any one of claims 1 to 9,
- in a second part, an aqueous acidic solution as employed in step (ii) of the process defined in any one of claims 1 to 9,
- in an optional third part means for mixing the content of the first and second parts, and, optionally, means for heating the resulting mixture, and
- in an optional fourth part, a substrate for receiving an optionally heated mixture of the content of said first and second parts and, optionally, means for transferring said optionally heated mixture to the substrate;

or, alternatively,

- in a first part, a sol material obtainable by the process consisting of steps (i) and (ii) as defined in any one of claims 1 to 9, and
- in a second part, a substrate for receiving said sol material and, optionally, means for transferring said sol material to said substrate.

15. A composition that is a mixture comprising silicon-based sol-gel precursors comprising a compound of formula $Si(OR^1)(OR^2)(OR^3)(OR^4)$ wherein each of $R^1$, $R^2$, $R^3$ and $R^4$ is independently a $(C_1\text{-}C_4)$alkyl chain, and a compound of formula $Si(R^5)(OR^6)(OR^7)(OR^8)$ wherein each of $R^5$, $R^6$, $R^7$ and $R^8$ is independently a $(C_1\text{-}C_4)$alkyl chain, the molar ratio compound of formula $Si(OR^1)(OR^2)(OR^3)(OR^4)$ : compound of formula $Si(R^5)(OR^6)(OR^7)(OR^8)$ being from 80:20 to 50:50, the mixture further comprising octenidine in an amount of from 1 to 7.5 grams per each 100 grams of the silicon-based sol-gel precursors.

**Patentansprüche**

1. Verfahren zur Herstellung einer Zusammensetzung, umfassend die Schritte des:

   (i) Bereitstellens eines Gemischs, das Silicon-basierte Sol-Gel-Vorläufer umfasst, umfassend eine Verbindung der Formel $Si(OR^1)(OR^2)(OR^3)(OR^4)$, wobei jedes von $R^1$, $R^2$, $R^3$ und $R^4$ unabhängig eine $(C_1\text{-}C_4)$alkyl-Kette ist, und eine Verbindung der Formel $Si(R^5)(OR^6)(OR^7)(OR^8)$, wobei jedes von $R^5$, $R^6$, $R^7$ und $R^8$ unabhängig eine $(C_1\text{-}C_4)$alkyl-Kette ist, wobei das Molverhältnis der Verbindung der Formel $Si(OR^1)(OR^2)(OR^3)(OR^4)$ : Verbindung der Formel $Si(R^5)(OR^6)(OR^7)(OR^8)$ bei von 80:20 bis 50:50 liegt, wobei das Gemisch des Weiteren Octenidin in einer Menge von 1 bis 7,5 g pro 100 g der Silicon-basierten Sol-Gel-Vorläufer umfasst;
   (ii) Behandelns des in Schritt (i) bereitgestellten Gemischs mit einer wässrigen sauren Lösung, um ein Sol-Material zu bilden;
   (iii) gegebenenfalls Aushärten des Sol-Materials aus Schritt (ii) durch Erhitzen des Materials bei einer Temperatur, die etwa Mundtemperatur entspricht.

2. Verfahren nach Anspruch 1, wobei die Verbindung der Formel $Si(OR^1)(OR^2)(OR^3)(OR^4)$ Tetraethyl-Orthosilicat und die Verbindung der Formel $Si(R^5)(OR^6)(OR^7)(OR^8)$ Methyltrimethoxysilan ist.

3. Verfahren nach einem der Ansprüche 1 bis 2, wobei das Molverhältnis der Verbindung der Formel $Si(OR^1)(OR^2)(OR^3)(OR^4)$ zur Verbindung der Formel $Si(R^5)(OR^6)(OR^7)(OR^8)$ bei etwa 60:40 liegt.

4. Verfahren nach einem der Ansprüche 1 bis 3, wobei die Silicon-basierten Sol-Gel-Vorläufer des Gemischs aus Schritt (i) die Verbindung der Formel $Si(OR^1)(OR^2)(OR^3)(OR^4)$ und die Verbindung der Formel $Si(R^5)(OR^6)(OR^7)(OR^8)$ in einer Menge von mindestens 80 Gew.-% in Bezug auf das Gesamtgewicht der Silicon-basierten Sol-Gel-Vorläufer umfassen.

5. Verfahren nach einem der Ansprüche 1 bis 4, wobei die Silicon-basierten Sol-Gel-Vorläufer des Gemischs aus Schritt (i) kein Glycidoxypropyltrimethoxysilan umfassen.

6. Verfahren nach einem der Ansprüche 1 bis 5, wobei das Gemisch aus Schritt (i) Octenidin in einer Menge von 1 bis 5 g pro 100 g der Silicon-basierten Sol-Gel-Vorläufer umfasst.

7. Verfahren nach einem der Ansprüche 1 bis 6, wobei die wässrige saure Lösung aus Schritt (ii) eine Salpetersäure-Lösung in einer Konzentration von 0,01 N bis 0,5 N ist.

8. Verfahren nach einem der Ansprüche 1 bis 7, wobei Schritt (ii) bei einer Temperatur von zwischen 50°C und 100°C durchgeführt wird.

9. Verfahren nach einem der Ansprüche 1 bis 8, wobei Schritt (iii) durchgeführt wird.

10. Zusammensetzung, die durch das in einem der Ansprüche 1 bis 9 definierten Verfahren erhältlich ist.

11. Zusammensetzung nach Anspruch 10 zur Verwendung in der Medizin.

12. Zusammensetzung nach Anspruch 10 zur Verwendung bei der Vorbeugung oder Behandlung von Entzündungsprozessen.

13. Zusammensetzung zur Verwendung nach Anspruch 12, wobei der Entzündungsprozess Periimplantitis ist.

14. Kit-of-parts zur Herstellung eines Substrats, das mit einer anti-infektiösen Beschichtungszusammensetzung beschichtet ist, umfassend:

- in einem ersten Teil, ein wie in Schritt (i) des in einem der Ansprüche 1 bis 9 definierten Verfahrens bereitgestelltem Gemisch,
- in einem zweiten Teil, eine wie in Schritt (ii) des in einem der Ansprüche 1 bis 9 definierten Verfahrens verwendete wässrige saure Lösung,
- in einem optionalen dritten Teil, Mittel zum Mischen des Inhalts des ersten und zweiten Teils und gegebenenfalls Mittel zum Erhitzen des resultierenden Gemischs, und
- in einem optionalen vierten Teil, ein Substrat zur Aufnahme eines gegebenenfalls erhitzen Gemischs des Inhalts des genannten ersten und zweiten Teils und gegebenenfalls Mittel zum Übertragen des genannten gegebenenfalls erhitzten Gemischs auf das Substrat;

oder, alternativ

- in einem ersten Teil, Sol-Material, das durch das aus den Schritten (i) und (ii) bestehenden Verfahren, wie in einem der Ansprüche 1 bis 9 definiert, erhältlich ist, und
- in einem zweiten Teil, ein Substrat zur Aufnahme des Sol-Materials und gegebenenfalls Mittel zum Übertragen des Sol-Materials auf das Substrat.

**15.** Zusammensetzung, die ein Gemisch ist, das Silicon-basierte Sol-Gel-Vorläufer umfasst, die eine Verbindung der Formel $Si(OR^1)(OR^2)(OR^3)(OR^4)$ umfassen, wobei jedes von $R^1$, $R^2$, $R^3$ und $R^4$ unabhängig eine $(C_1-C_4)$alkyl-Kette ist, und eine Verbindung der Formel $Si(R^5)(OR^6)(OR^7)(OR^8)$, wobei jedes von $R^5$, $R^6$, $R^7$ und $R^8$ unabhängig eine $(C_1-C_4)$Alkylkette ist, wobei das Molverhältnis der Verbindung der Formel $Si(OR^1)(OR^2)(OR^3)(OR^4)$ : Verbindung der Formel $Si(R^5)(OR^6)(OR^7)(OR^8)$ bei von 80:20 bis 50:50 liegt, wobei das Gemisch des Weiteren Octenidin in einer Menge von 1 bis 7,5 g pro 100 g der Silicon-basierten Sol-Gel-Vorläufer umfasst.

## Revendications

**1.** Procédé de préparation d'une composition comprenant les étapes suivantes :

(i) la fourniture d'un mélange comprenant des précurseurs de sol-gel à base de silicium, comprenant un composé de formule $Si(OR^1)(OR^2)(OR^3)(O^{R4})$ dans laquelle chacun parmi $R^1$, $R^2$, $R^3$ et $R^4$ est indépendamment une chaîne alkyle en $(C_1-C_4)$, et un composé de formule $Si(R^5)(OR^6)(OR^7)(OR8)$ dans laquelle chacun parmi $R^5$, $R^6$, $R^7$ et $R^8$ est indépendamment une chaîne alkyle en $(C_1-C_4)$, le rapport molaire composé de formule $Si(OR^1)(OR^2)(OR^3)(OR^4)$:composé de formule $Si(R^5)(OR^6)(OR^7)(OR^8)$ étant de 80:20 à 50:50, le mélange comprenant en outre de l'octénidine en une quantité de 1 à 7,5 grammes pour 100 grammes des précurseurs de sol-gel à base de silicium ;
(ii) le traitement du mélange fourni dans l'étape (i) avec une solution acide aqueuse afin de former un matériau de sol ;
(iii) facultativement, le durcissement du matériau de sol de l'étape (ii) en chauffant ledit matériau à une température égale à environ la température buccale.

**2.** Procédé selon la revendication 1, dans lequel le composé de formule $Si(OR^1)(OR^2)(OR^3)(O^{R4})$ est l'orthosilicate de tétraéthyle et le composé de formule $Si(R^5)(OR^6)(OR^7)(OR^8)$ est le méthyltriméthoxysilane.

**3.** Procédé selon quelconque des revendications 1 à 2, dans lequel le rapport molaire du composé de formule $Si(OR^1)(OR^2)(OR^3)(OR^4)$ par rapport au composé de formule $Si(R^5)(OR^6)(OR^7)(OR^8)$ est d'environ 60:40.

**4.** Procédé selon quelconque des revendications 1 à 3, dans lequel les précurseurs de sol-gel à base de silicium du mélange de l'étape (i) comprennent le composé de formule $Si(OR^1)(OR^2)(OR^3)(O^{R4})$ et le composé de formule $Si(R^5)(OR^6)(OR^7)(OR^8)$ en une quantité d'au moins 80 % en poids par rapport au poids total des précurseurs de sol-gel à base de silicium.

**5.** Procédé selon quelconque des revendications 1 à 4 dans lequel les précurseurs de sol-gel à base de silicium du mélange de l'étape (i) ne comprennent pas de glycidoxypropyltriméthoxysilane.

**6.** Procédé selon quelconque des revendications 1 à 5, dans lequel le mélange de l'étape (i) comprend de l'octénidine en

une quantité de 1 à 5 grammes pour 100 grammes des précurseurs de sol-gel à base de silicium.

**7.** Procédé selon quelconque des revendications 1 à 6, dans lequel la solution acide aqueuse de l'étape (ii) est une solution d'acide nitrique à une concentration de 0,01 N à 0,5 N.

**8.** Procédé selon quelconque des revendications 1 à 7, dans lequel l'étape (ii) est réalisée à une température entre 50 °C et 100 °C.

**9.** Procédé selon quelconque des revendications 1 à 8 dans lequel l'étape (iii) est réalisée.

**10.** Composition pouvant être obtenue par le procédé tel que défini dans quelconque des revendications 1 à 9.

**11.** Composition selon la revendication 10 pour une utilisation en médecine.

**12.** Composition selon la revendication 10 pour une utilisation dans la prévention ou le traitement des processus inflammatoires.

**13.** Composition pour une utilisation selon la revendication 12 dans laquelle ledit processus inflammatoire est une péri-implantite.

**14.** Kit de parties pour la préparation d'un substrat revêtu d'une composition de revêtement anti-infectieux comprenant :

- dans une première partie, un mélange tel que fourni dans l'étape (i) du procédé tel que défini dans quelconque des revendications 1 à 9,
- dans une deuxième partie, une solution acide aqueuse telle qu'utilisée dans l'étape (ii) du procédé tel que défini dans quelconque des revendications 1 à 9,
- dans une troisième partie facultative, un moyen pour mélanger le contenu des première et deuxième parties et, facultativement, un moyen pour chauffer le mélange résultant, et
- dans une quatrième partie facultative, un substrat pour recevoir un mélange facultativement chauffé du contenu desdites première et deuxième parties et, facultativement, un moyen pour transférer ledit mélange facultativement chauffé sur le substrat ;

ou, alternativement,

- dans une première partie, un matériau de sol pouvant être obtenu par le procédé consistant en les étapes (i) et (ii) tel que défini dans quelconque des revendications 1 à 9, et
- dans une seconde partie, un substrat pour recevoir ledit matériau de sol et, facultativement, un moyen pour transférer ledit matériau de sol sur ledit substrat.

**15.** Composition qui est un mélange comprenant des précurseurs de sol-gel à base de silicium, comprenant un composé de formule $Si(OR^1)(OR^2)(OR^3)(OR^4)$ dans laquelle chacun parmi $R^1$, $R^2$, $R^3$ et $R^4$ est indépendamment une chaîne alkyle en ($C_1$-$C_4$), et un composé de formule $Si(R^5)(OR^6)(OR^7)(OR^8)$ dans laquelle chacun parmi $R^5$, $R^6$, $R^7$ et $R^8$ est indépendamment une chaîne alkyle en ($C_1$-$C_4$), le rapport molaire composé de formule $Si(OR^1)(OR^2)(OR^3)(OR^4)$:composé de formule $Si(R^5)(OR^6)(OR^7)(OR^8)$ étant de 80:20 à 50:50, le mélange comprenant en outre de l'octénidine en une quantité de 1 à 7,5 grammes pour 100 grammes des précurseurs de sol-gel à base de silicium.

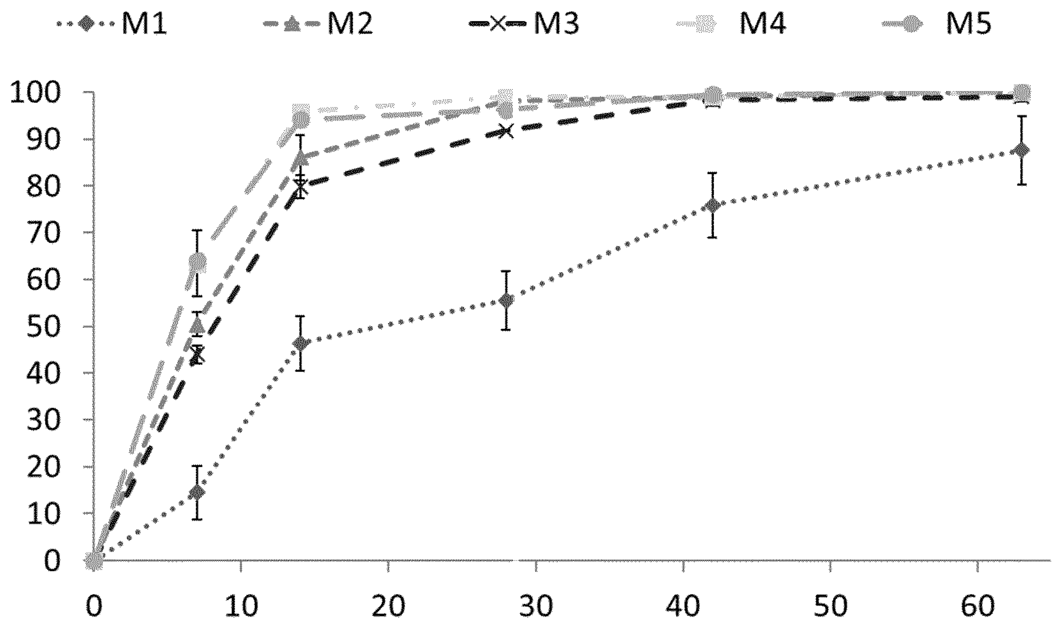
M1
M2
M3
M4
M5
100
90
80
70
60
50
40
30
20
10
0
0
10
20
30
40
50
60

FIG. 1

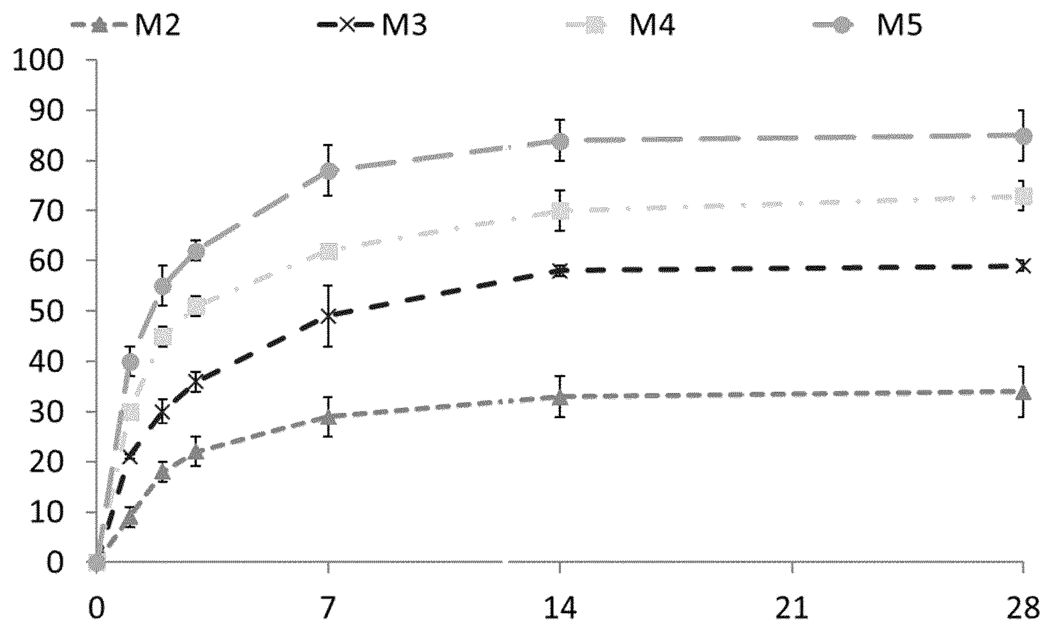
M2
M3
M4
M5
100
90
80
70
60
50
40
30
20
10
0
0
7
14
21
28

FIG. 2

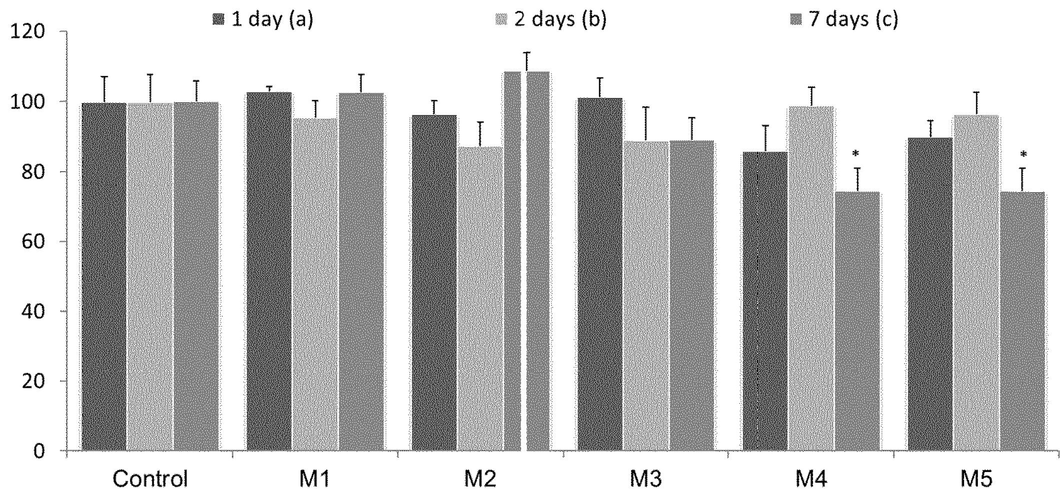


FIG. 3

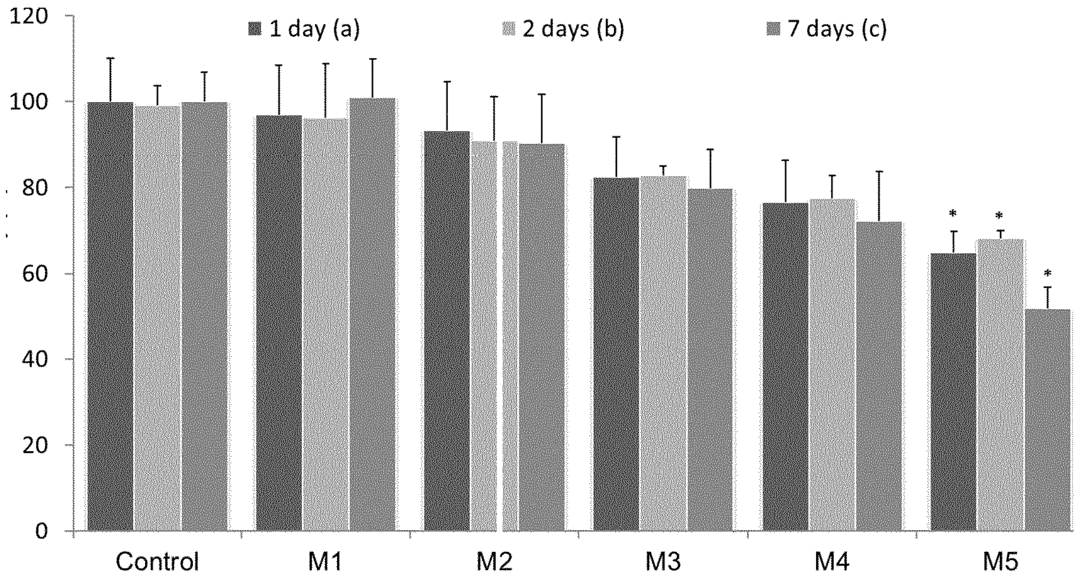


FIG. 4

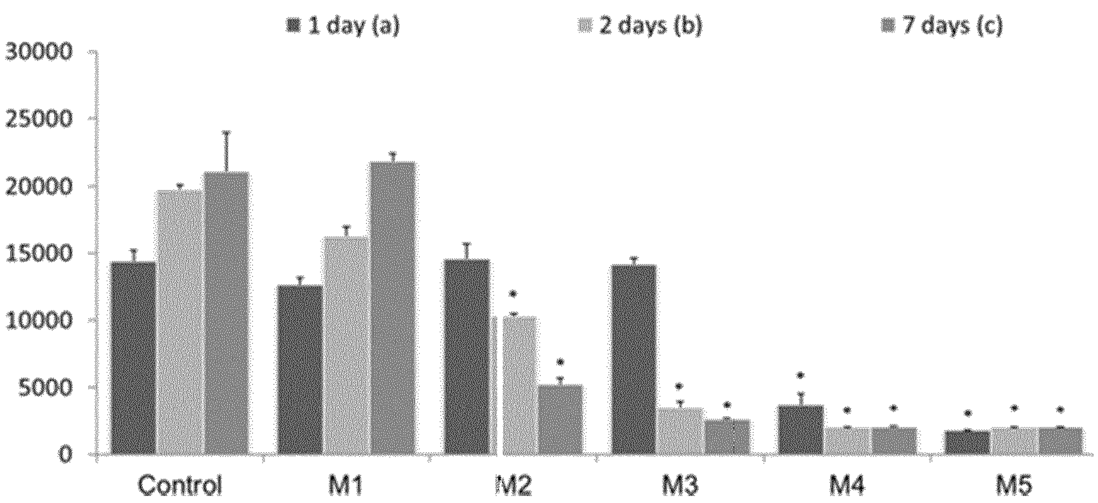


FIG 5

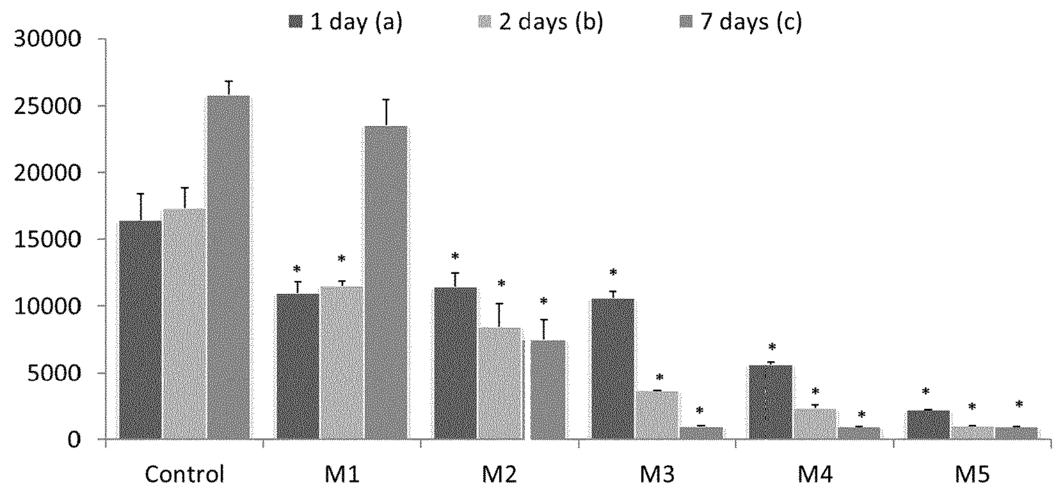
1 day (a)
2 days (b)
7 days (c)
30000
25000
20000
15000
10000
5000
0
Control
M1
M2
M3
M4
M5

FIG. 6

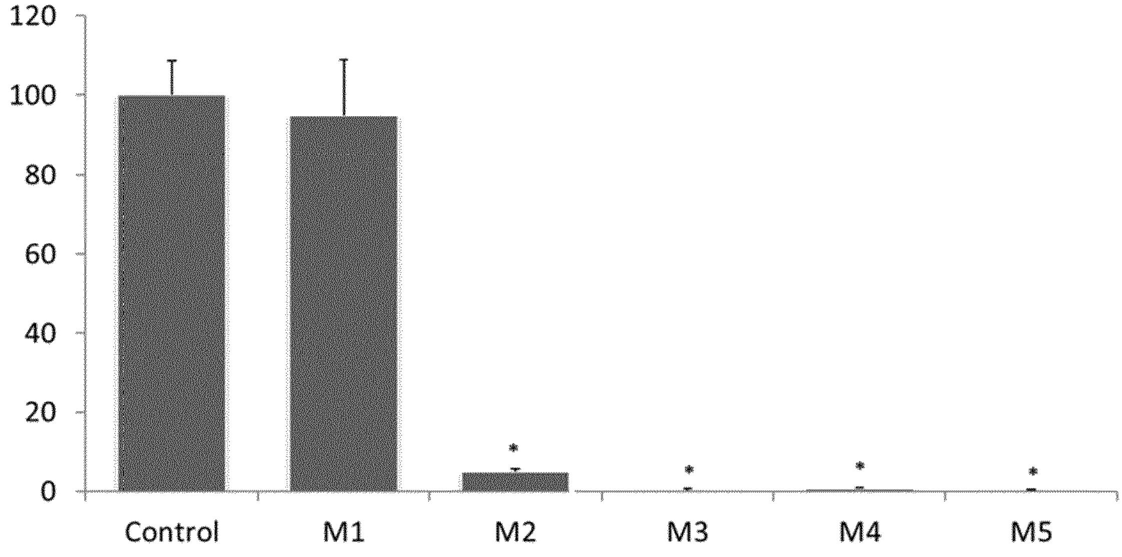
120
100
80
60
40
20
0
Control
M1
M2
M3
M4
M5

FIG. 7

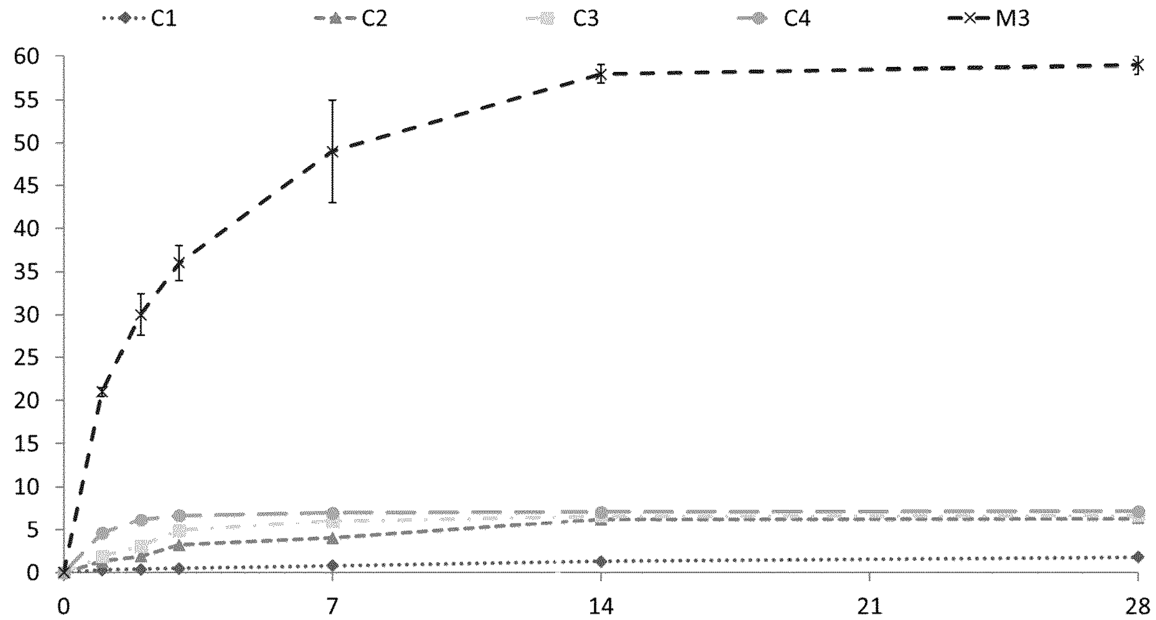


FIG. 8

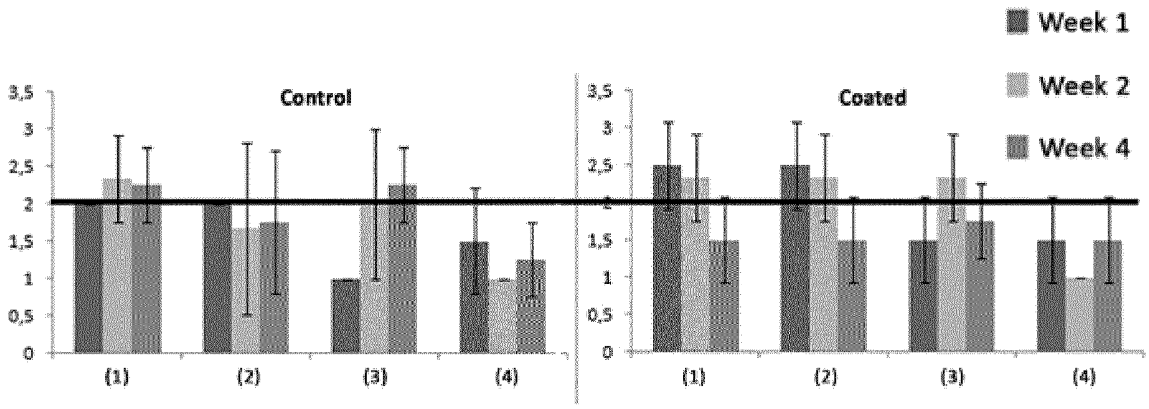


FIG. 9

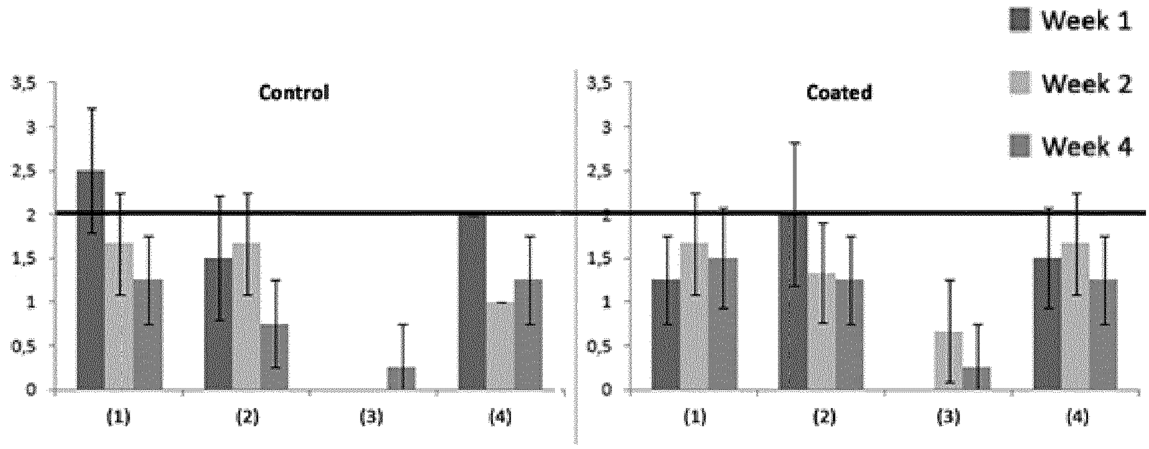


FIG. 10

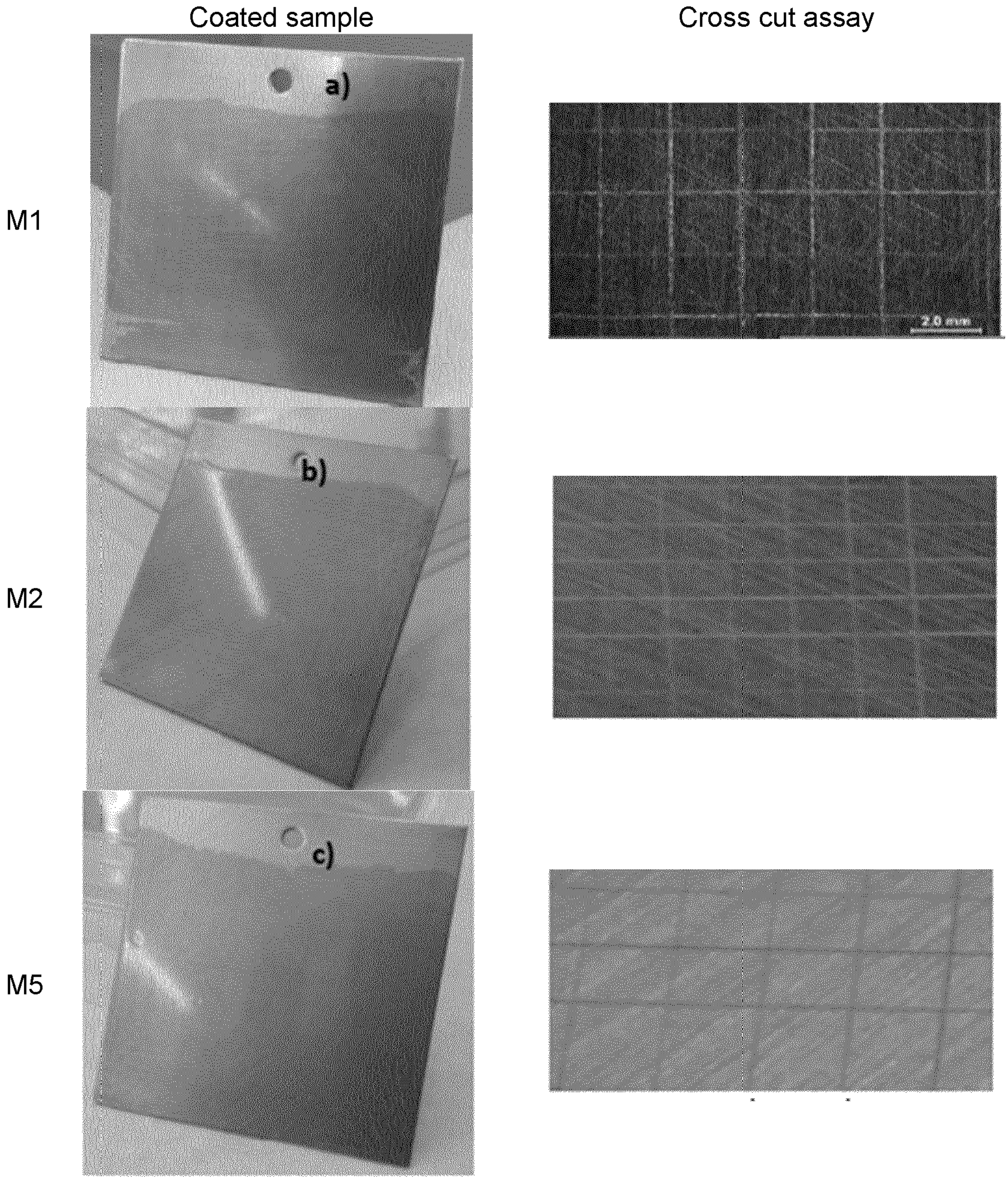


FIG. 11

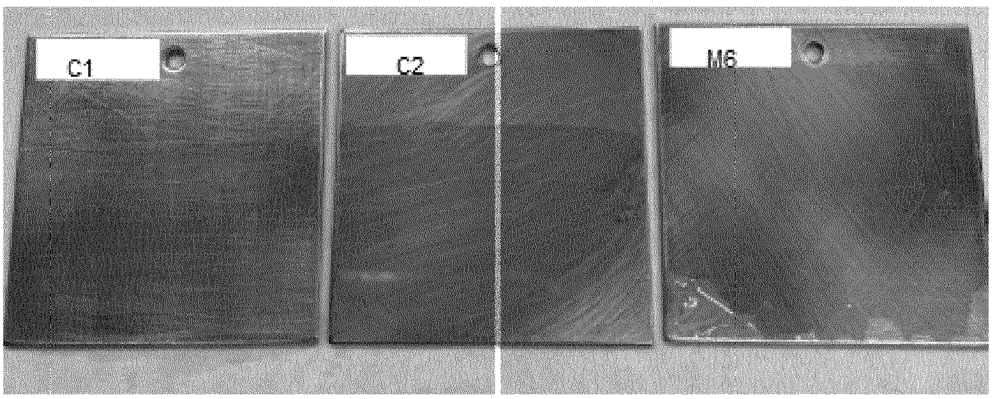


FIG. 12

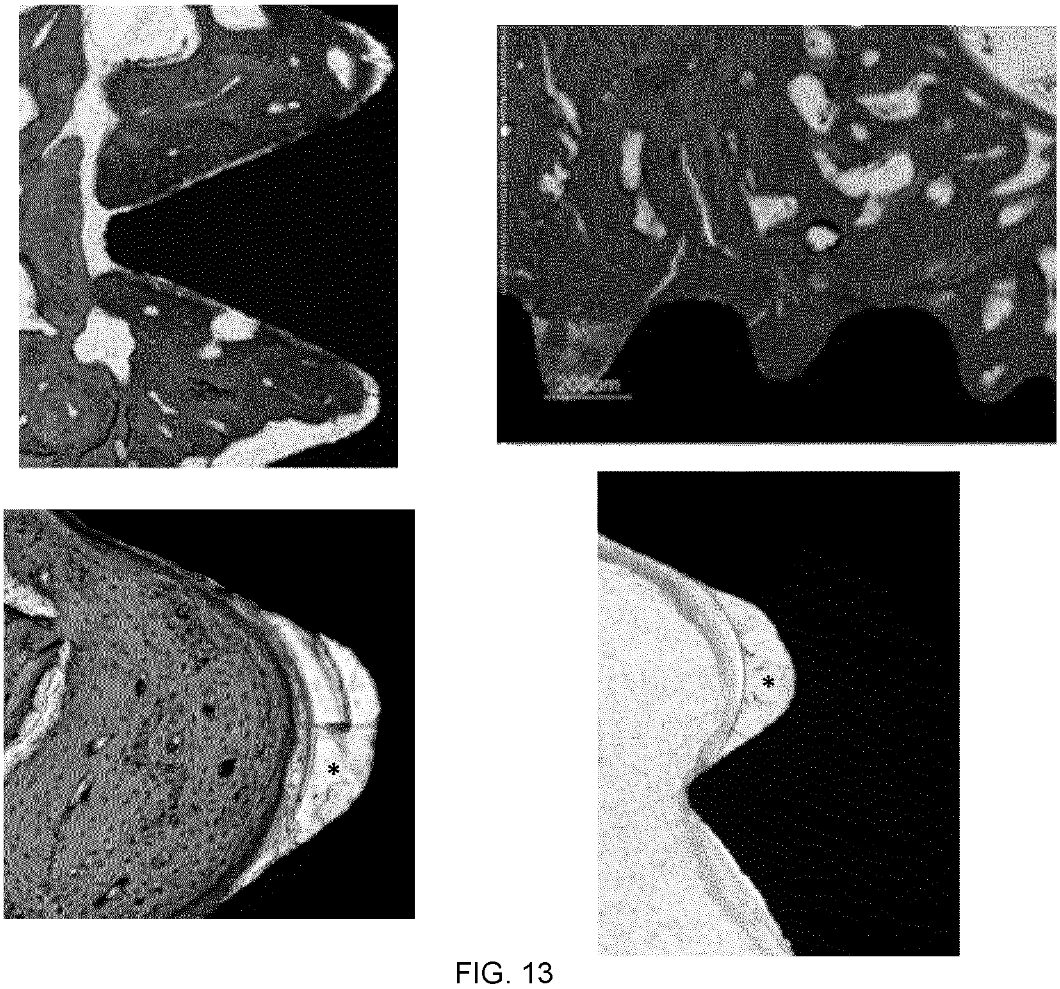

FIG. 13

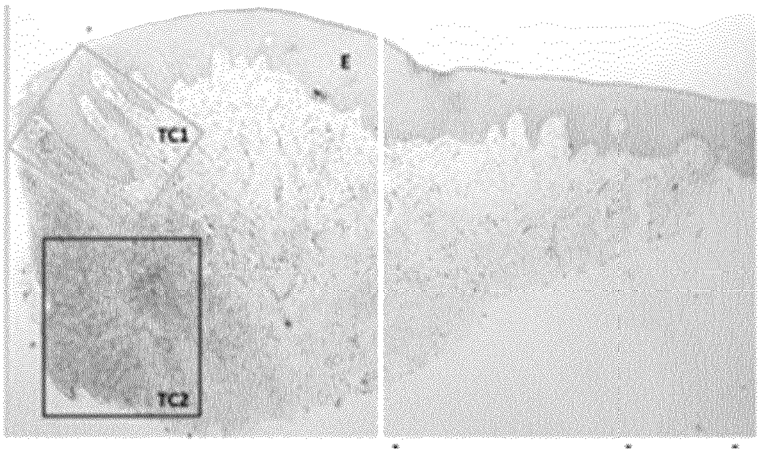


FIG. 14

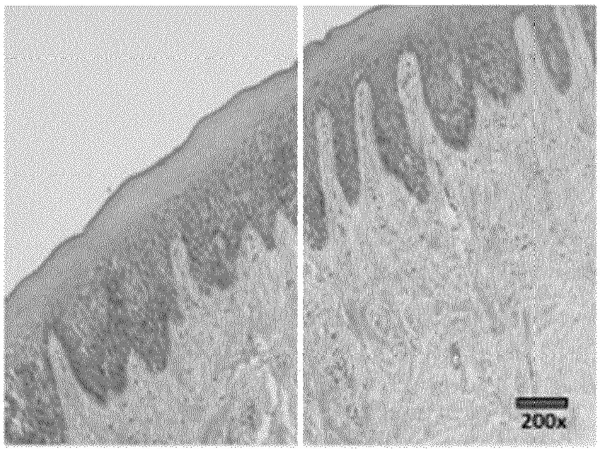


FIG. 15

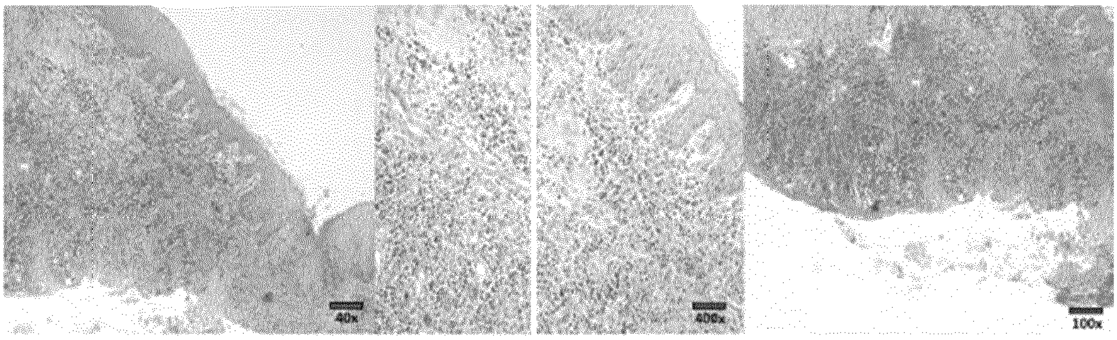


FIG. 16

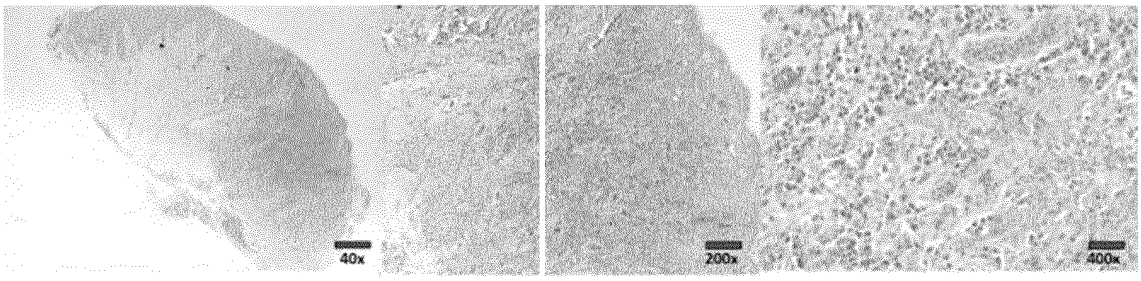


FIG. 17

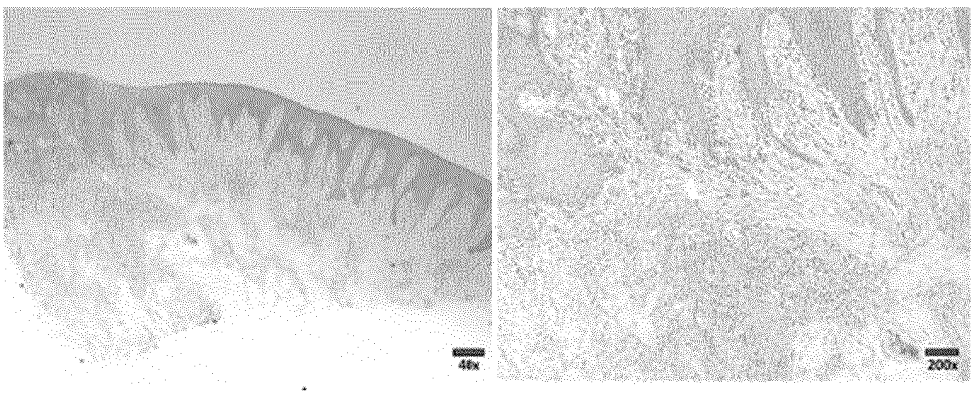


FIG. 18

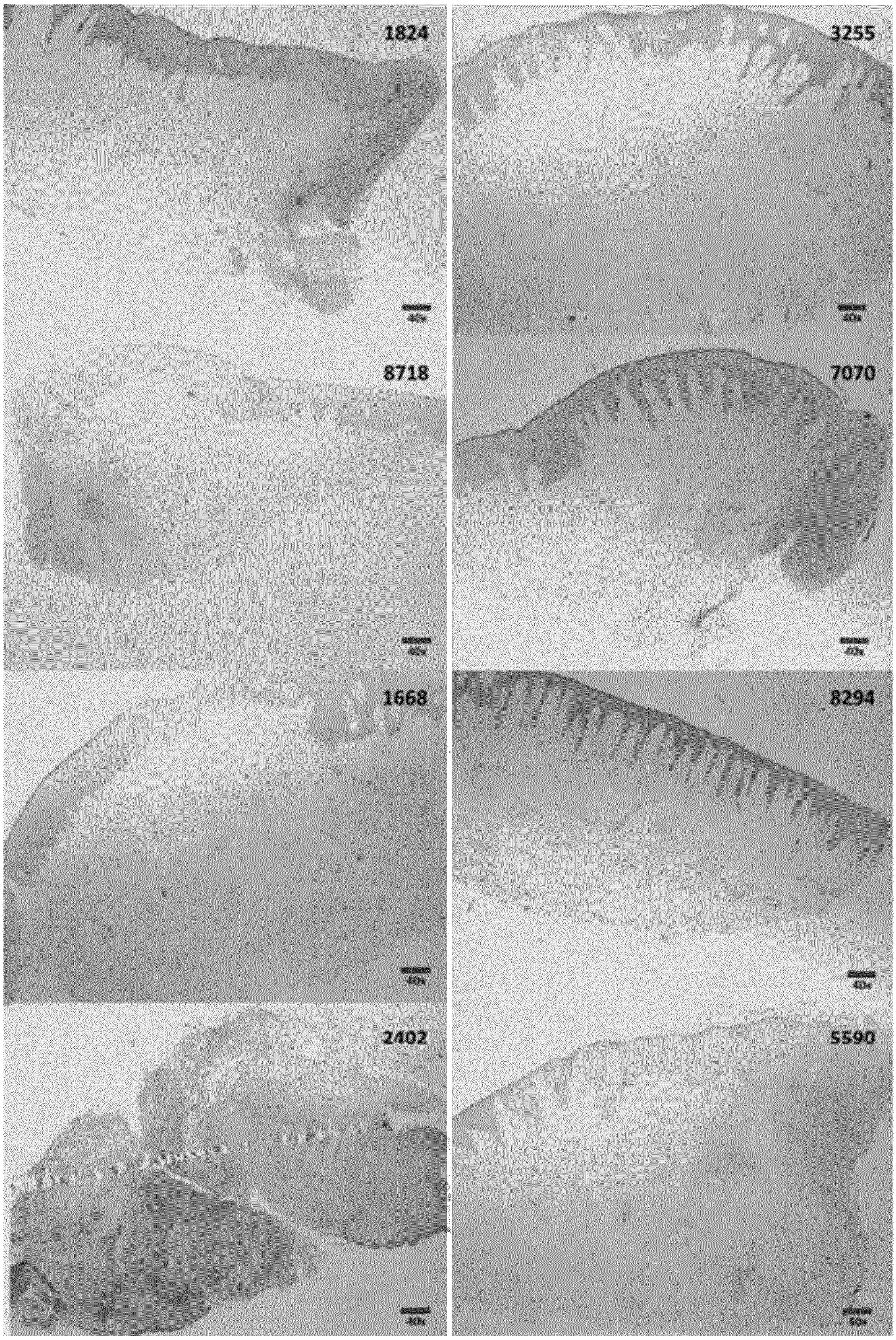
1824
40x
3255
40x
8718
40x
7070
40x
1668
40x
8294
40x
2402
40x
5590
40x

FIG. 19

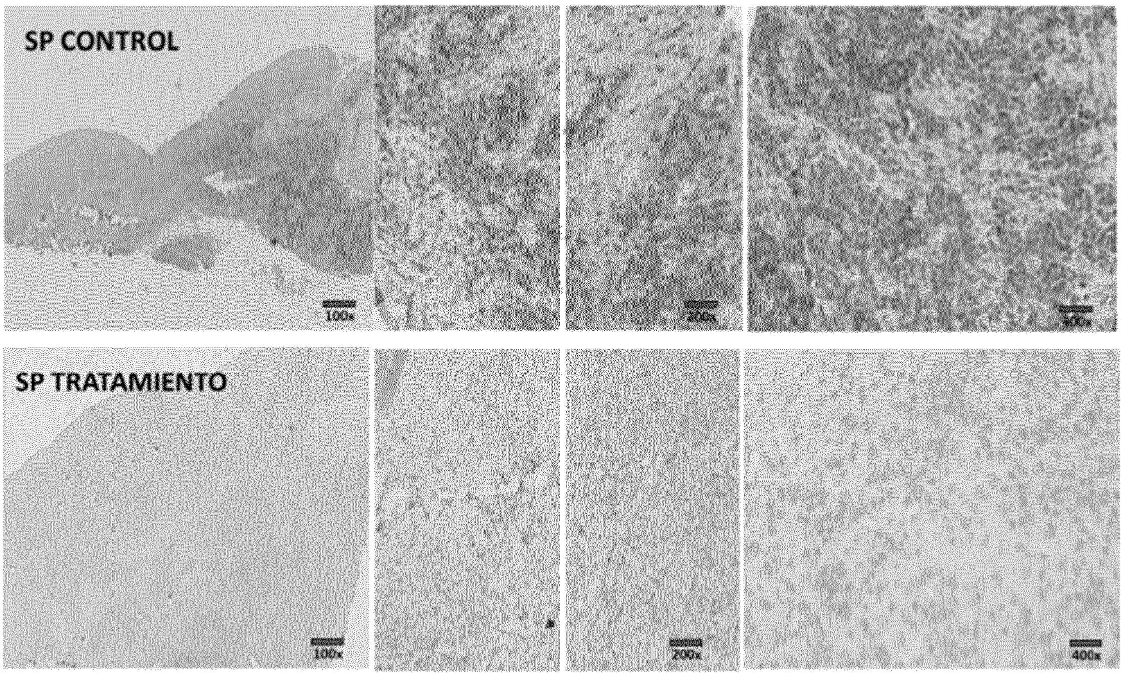


FIG. 20

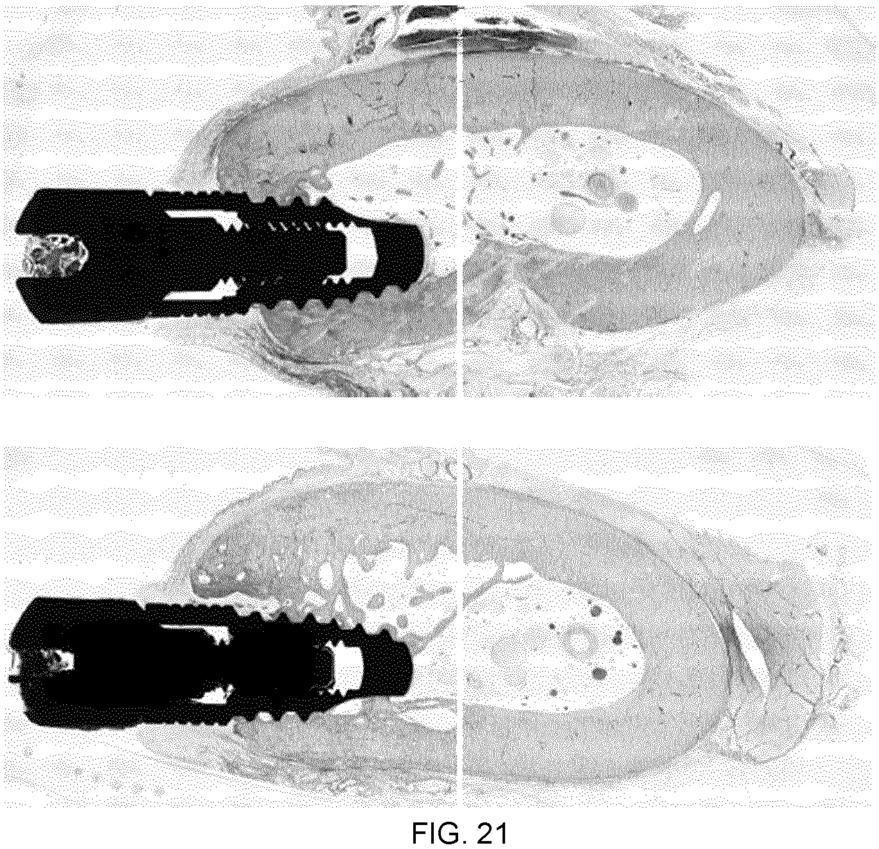

FIG. 21

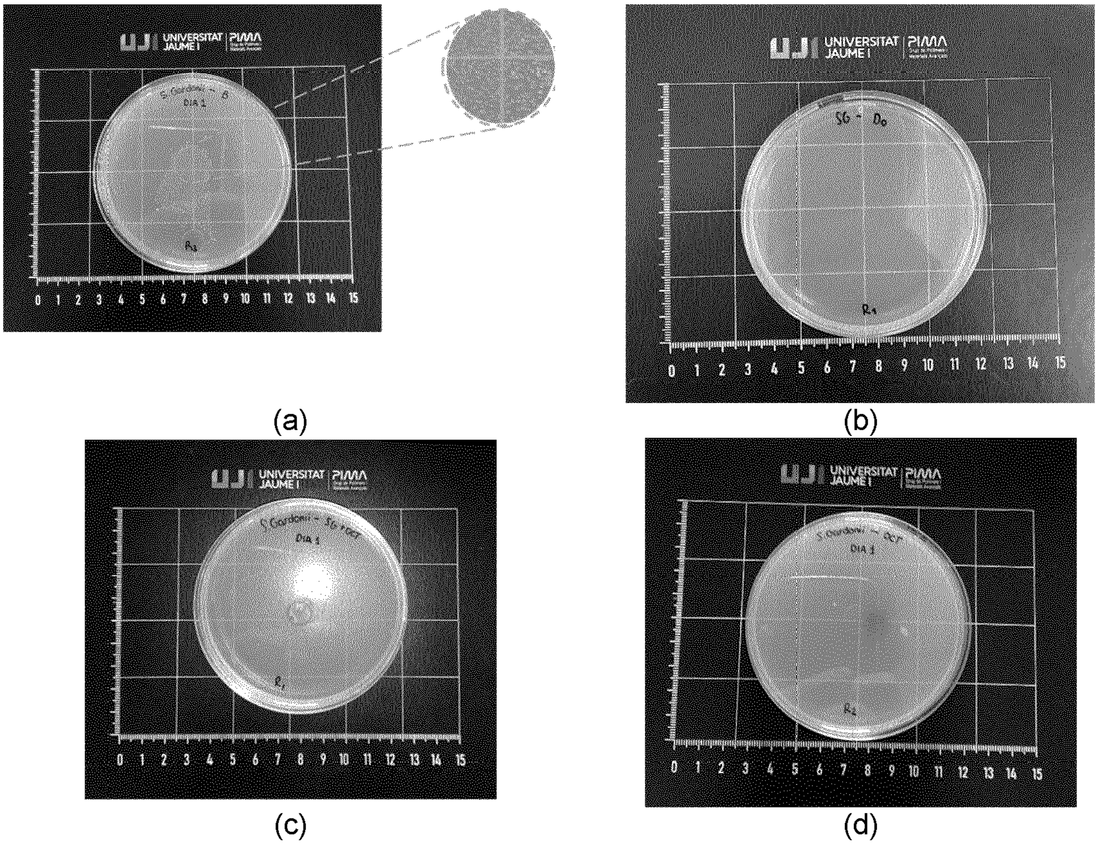


FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1799186 B1
- WO 2017197510 A1
- ES 201031831

### Non-patent literature cited in the description

- **RADIN** ; **DUCHEYNE**. *Biomaterials*, 2007, vol. 28, 1721-1729
- **I. GARCÍA-ARNÁEZ** ; **B. PALLA** ; **J. SUAY** ; **F. ROMERO-GAVILÁN** ; **L. GARCÍA-FERNÁNDEZ** ; **M. FERNÁNDEZ** ; **I. GOÑI** ; **M. GURRUCHAGA**. *European Polymer Journal*, 2019, vol. 113, 289-296
- **ROMERO-GAVILÁN et al.** *Biofouling*, 2017, vol. 33 (8), 676-689
- **DONATH** ; **BREUNER**. *J. Oral Pathol. Med.*, 1982, vol. 11, 318-326